(19)

Europäisches
Patentamt
European
Patent Office
Office européen
des brevets

(11) **EP 4 108 678 A1**

(12) **EUROPEAN PATENT APPLICATION**
published in accordance with Art. 153(4) EPC

(43) Date of publication:
**28.12.2022 Bulletin 2022/52**

(21) Application number: **21756429.3**

(22) Date of filing: **18.02.2021**

(51) International Patent Classification (IPC):
*C07K 14/00* (2006.01)      *C12M 1/34* (2006.01)
*C12N 5/10* (2006.01)       *C12N 1/15* (2006.01)
*C12N 1/19* (2006.01)       *C12N 1/21* (2006.01)
*C12N 15/53* (2006.01)      *C12Q 1/00* (2006.01)
*C12Q 1/32* (2006.01)       *C12N 9/04* (2006.01)
*G01N 33/52* (2006.01)      *C12P 21/02* (2006.01)

(52) Cooperative Patent Classification (CPC):
**C07K 14/00; C12M 1/34; C12N 5/10; C12N 9/0004;
C12N 15/74; C12N 15/80; C12N 15/81;
C12P 21/02; C12Q 1/00; C12Q 1/32; G01N 33/52**

(86) International application number:
**PCT/JP2021/006153**

(87) International publication number:
**WO 2021/167011 (26.08.2021 Gazette 2021/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.02.2020   JP 2020028722
19.06.2020   JP 2020106467**

(71) Applicant: **Kikkoman Corporation
Noda-shi, Chiba 278-8601 (JP)**

(72) Inventors:
 • **MASAKARI Yosuke
   Noda-shi, Chiba 278-8601 (JP)**
 • **INAMOTO Chiaki
   Noda-shi, Chiba 278-8601 (JP)**
 • **NAKAMURA Miki
   Noda-shi, Chiba 278-8601 (JP)**
 • **KAN Eiichiro
   Noda-shi, Chiba 278-8601 (JP)**
 • **ICHIYANAGI Atsushi
   Noda-shi, Chiba 278-8601 (JP)**
 • **HIRAGUCHI Haruka
   Noda-shi, Chiba 278-8601 (JP)**
 • **SUZUKI Ryota
   Noda-shi, Chiba 278-8601 (JP)**

(74) Representative: **J A Kemp LLP
80 Turnmill Street
London EC1M 5QU (GB)**

Remarks:
The complete document including Reference Table(s) and the Sequence Listing(s) can be downloaded from the EPO website

(54) **DEVICE FOR EVALUATING STATE OF SAMPLE, SYSTEM INCLUDING SAME, METHOD FOR EVALUATING STATE OF SAMPLE, AND LACTATE DEHYDROGENASE USED THEREFOR**

(57)    An object of the present invention is to provide a device for evaluating a state of interstitial fluid, blood, urine, tears, sweat, saliva of human and non-human organisms, foods and drinks, brewed product, etc., a system, a program, and an evaluation method using these. The present invention is to provide a device for evaluating a state of a sample which comprises an action part for allowing lactate dehydrogenase to act on a sample, and a sensor for sensing the state of the sample on which lactate dehydrogenase is allowed to act, a system, a program, a method for evaluating the state of the sample using these, and an enzyme to be used therefor.

EP 4 108 678 A1

**Description**

TECHNICAL FIELD

The present invention relates to a device for evaluating a state of a sample containing a sensor using a flavin-dependent lactate dehydrogenase which uses a flavin compound as a coenzyme, a system in which the above-mentioned device further contains an output part, and a method for evaluating a state of a sample, which uses the above-mentioned device or system. Also, the present invention relates to a flavin-dependent lactate dehydrogenase that can be suitably used for the devices, systems and methods for evaluating a state of sample using them.

BACKGROUND ART

Blood lactate concentration and sweat lactate concentration have been known as markers that reflect fatigue and physical conditions. Lactic acid is a principal metabolite and is recognized to be important not only for an index of management of health but also for health assessments including serious illness and/or surgical patients, and lactic acid value in body fluids can be an index for various pathological conditions such as circulatory failure, hepatic disorder, etc. Monitoring of lactic acid can be used for detecting sepsis, hypoxia, and the presence of cancer tissue (Non-Patent Document 1).

For the purpose of management of physical conditions not limited to a human in diseased state, monitoring of lactic acid is carried out as one of indexes for monitoring appropriateness of training conducted by athletes and those who are highly interested in exercising on a daily basis (Non-Patent Document 2).

In Patent Documents 1 to 3, as technical proposals for continuously monitoring a human condition, posture, etc., for a certain period of time, predetermined information processing devices have been proposed (Patent Documents 1 to 3).

As an enzyme using lactic acid as a substrate, lactate oxidase (hereinafter referred to as LOD) has been known. LOD involves a problem that its activity is lost during storage after drying. Therefore, in Patent Document 4, a method of drying in combination with a specific stabilizing agent has been proposed in the drying step.

In Patent Document 5, as a means for avoiding lowering in LOD activity by hydrogen peroxide, a lactic acid sensor in which catalase coexists for the purpose of eliminating hydrogen peroxide has been proposed.

Also, as an enzyme using lactic acid as a substrate, FMN-dependent lactate dehydrogenase (hereinafter, FMN-LDH) has been known. In Non-Patent Document 3, there is described that the previously known Saccharomyces cerevisiae-derived FMN-LDH involves a problem in stability.

PRIOR ART DOCUMENTS

PATENT DOCUMENTS

Patent Document 1: JP 2019-150649A
Patent Document 2: JP 2017-100039A
Patent Document 3: WO 17/163521
Patent Document 4: JP Patent No. 5,593,689
Patent Document 5: JP 2018-519507A

NON-PATENT DOCUMENT

Non-Patent Document 1: The Japanese Clinical Practice Guidelines for Management of Sepsis and Septic Shock 2016, PNAS September 15, 2015, 112 (37), 11642-11647

Non-Patent Document 2: Research journal of sports performance, 3, 31-48, 2011

Non-Patent Document 3: Methods Enzymol., 53, 238-56, 1978

SUMMARY OF THE INVENTION

PROBLEMS TO BE SOLVED BY THE INVENTION

The lactic acid value in foods can be an index for quality control, etc. In a manufacturing floor of various kinds of fermented foods and drinks produced through a lactic acid fermentation process, for example, Japanese rice wine or wine, whisky, cheese, yoghurt, sauerkraut, kuzumochi, pickles, fermented soybean paste (miso), soy sauce, lactic acid fermented yeast extract, etc., it is preferable to carry out a suitable management of the lactic acid fermentation processing in order to produce a good quality product stably, and in the management of the processing, the amount of lactic acid can be an index of the management of the production processing.

Further, formation of lactic acid is not limited only to foods and drinks fermented with lactic acid but also, for example, progressed in lactic acid fermentation compositions obtained by fermenting vegetable materials which are raw materials for cosmetics with lactic acid bacteria, and in lactic acid compositions produced as chemical products, and the amount of lactic acid can be an index in the management of production process and quality control of such products.

In addition, an oligomer or polymer containing lactic acid, for example, when polylactic acid is decomposed, the amount of lactic acid of the monomer can be an index for calculating the decomposition ratio.

It is preferable that measurement of an amount of lactic acid carried out for the above-mentioned purpose can be carried out frequently and simple and easy in dairy life or in the producing process of foods and drinks, etc., every time, is more preferable that the measurement can be carried out repeatedly in the possible simplest process, and is further preferable to be able to monitor automatically and continuously.

At present, no device is present for measuring lactic acid capable of monitoring an amount of lactic acid in a sample simple and easily, and continuously, and it is the present status that a sample (for example, blood collected from the body of the patient by puncture) which might contain lactic acid obtained from the sample through some pretreatment is measured by a means each time by some kind of method.

Invasive sampling methods such as puncture, etc., in the current measurement method cause pain or stress for biological samples, which is hindrance of subjecting to frequent sampling. Also, in the management of production process such as foods and drinks, etc., that do not have the problem of pain and stress, sampling is manually carried out from the lactic acid-containing composition during the production each time, then, lactic acid is measured, and the measurement data must be recorded manually each time, so that such frequent sampling operations in the current measurement technique of lactic acid is accompanied by complication.

As a technical proposal in which the state or posture, etc., of a human is continuously monitored for a certain period of time, for example, there are proposed a system in which a plurality of sensors are attached to a body or clothing of a human, a training machine, etc., and based on the certain living body-derived data obtained by the sensors and input information such as the age and gender, etc., of the human, a training program is proposed or a physical condition of the human is estimated, and an various information processing device for activating application programs based on the various kinds of data, etc. (Patent Documents 1 to 3). And in a small number of such proposals, there are some proposals including the words of lactic acid measurement. In those proposals, however, no method is disclosed that can specifically measure the amount of lactic acid as an actual index and can monitor the state of the sample thereby.

For example, in Patent Document 1, it has been proposed a system in which it can be attached to a training machine for riding a bicycle, and heartbeat information obtained from a human (driver) during the training time by a heartbeat meter is analyzed based on the heartbeat at the time of rest and the heartbeat during training of the driver, information such as age and gender, etc., and running data of a bicycle, etc., and output a message for bringing the current exercise state of the driver closer to the ideal state In Patent Document 2, it has been proposed a system in which respective blood flows at the first site where blood flow does not fluctuate depending on the physical condition of a human and at the second site where blood flow fluctuates depending on the physical condition are measured using non-contact camera technology such as a laser speckle camera, a laser Doppler blood flow system, etc., if necessary, based on the other individual information which can be additionally input or capable of being measured, sleepiness, alcohol-impairment, hungry level, motion sickness, stress level or depression level, etc., of a human are estimated. In Patent Document 3, it has been proposed an information processing device in which human body temperature, acceleration, heartbeat data, GPS data, height data, etc., are obtained using one or more wearable sensors attached to human underwear or outer wear, hats, eyeglasses, earplugs, headphone, etc., and estimation of the posture of the human wearing these, movements such as sleeping, waking up, walking, etc., and whether there is a sign of alpine disease during mountain climbing, etc., can be monitored. However, in any of the proposals, there is no disclosure that acquisition of lactic acid data can be simply and easily, and continuously realized similarly to acquisition of heartbeat measurement, blood flow measurement, GPS data, etc., in which simple and easy, and continuous data acquisition have already been technically realized, thereby monitoring of lactic acid and evaluation of the state of the human can be realized, nor technical proposals for realizing it.

In the technical problem for realizing simple and easy, and preferably continuous lactic acid monitoring, there may be mentioned a problem of performance issues of the enzyme for measurement in the enzyme method measurement as a means for measuring lactic acid.

As an enzyme using lactic acid as a substrate, lactate oxidase (hereinafter referred to as LOD), NAD-dependent lactate dehydrogenase (hereinafter referred to as NAD-LDH) using nicotinamide dinucleotide (NAD) as a coenzyme, and FMN-dependent lactate dehydrogenase (hereinafter referred to as FMN-LDH) using flavin mononucleotide (FMN) as a coenzyme have been known. A measurement device of lactic acid in blood using LOD is commercially available, and the stored sensor (electrode part for measuring lactic acid in blood containing LOD) is taken out at each time of the measurement and inserted into a dedicated measuring device, the blood squeezed from the human sample by puncture is sucked into the sensor and the measured value displayed on the measuring device is read. According to this, an amount of lactic acid in the sample can be known sporadically, and by repeating this, the amount of lactic acid in the sample can be measured each time

However, as mentioned above, when lactic acid in a lactic acid-containing composition containing biological interstitial fluid, blood and sweat, foods and drinks, and chemical products is considered to be measured stably for a long period of time, good accuracy, and preferably continuously, and simple and easy, known enzymes for measurement cannot correspond sufficiently.

LOD has a problem that thermal stability is not sufficient, and even when the LOD product is produced, the activity is lost to a large extent during the drying process or storage after drying. As a proposal for this problem, a method of drying in combination with a specific stabilizer in the drying process has been proposed (Patent Document 4), but the effect of improving the stability is still insufficient. It should be also considered that the stability of LOD in the liquid state during the actual enzymatic reaction is even lower than the stability in the powder state. For example, assuming a situation that monitoring of lactic acid is carried out for a long period of time in a scene where a sensor using LOD is used in close contact with the human body for a long period of time, or in a producing process of foods and drinks that can be placed under the fermentation temperature of a lactic acid bacterium fermentation processing, from the viewpoint of the stability of the enzyme from around room temperature to around 37°C, which is near human body temperature, there is a concern that the existing LOD cannot exhibit sufficient practicality.

Further, in LOD, there is a problem that it produces hydrogen peroxide as a reaction product. In particular, when the lactic acid sensor is in close contact with the skin or, in some cases, assuming the use of implanting it under the skin, there is concern that it is not preferable to adopt a sensor equipped with an enzyme which is capable of continuously generating hydrogen peroxide, which is a kind of the active oxygen species and the causative substance of oxidative stress. Further, hydrogen peroxide generated by the action of LOD exerts bad effect on the stabilization of LOD itself, and as a means for avoiding lowering in LOD activity thereby, a lactic acid sensor in which catalase coexists for the purpose of eliminating hydrogen peroxide has also been proposed (Patent Document 5).

NAD-LDH catalyzes an enzymatic reaction which does not produce hydrogen peroxide, so that no problem of producing hydrogen peroxide occurs. However, in the enzymatic reaction system using NAD-LDH, lactic acid and pyruvic acid reversibly react, so that there is a problem that accurate measured values cannot be obtained for the purpose of determination, and it is difficult to adopt it to the sensor.

FMN-LDH also catalyzes an enzymatic reaction which does not produce hydrogen peroxide so that the problem of producing hydrogen peroxide does not arise. Also, there is no problem of reversible reaction, so that among the above-mentioned three enzymes, it is considered to be the most promising practical enzyme for the purpose of monitoring lactic acid, but in Saccharomyces cerevisiae-derived FMN-LDH having being known conventionally, there is still a problem in stability (Non-Patent Document 3).

That is, as the technical problems for satisfying the market needs for simple and easy, and preferably continuous lactic acid monitoring in the evaluation of the state of the biological samples, management of production and quality control, etc., of foods and drinks, there exist problems in the above-mentioned sampling method, the problem of complicated recording processing associated with data processing for each single measurement, and further, the problem in the point of performance of the enzyme used for measuring lactic acid. An object of the present invention is to provide a device for monitoring or evaluating a state of a sample, a method for monitoring or evaluating a state of a sample and FMN-LDH used therefor, which can solve at least a part of the above-mentioned problems.

MEANS TO SOLVE THE PROBLEMS

The present inventors have conceived, in view of the above-mentioned problems, a device for evaluating a state of a sample having a constitution capable of simple and easy measuring lactic acid in a sample non-invasively when measuring an amount of lactic acid in the sample, and by using such a device, they have found a method for evaluating a state of a sample and a system used therefor, and FMN-LDH suitably applicable to the lactic acid measurement, whereby they have accomplished the present invention.

The present invention includes the following embodiments.

(1) A device for evaluating a state of a sample, which device comprises

an action part for allowing lactate dehydrogenase to act on a sample, and
a sensor for sensing the state of a sample on which lactate dehydrogenase is allowed to act, which sensor is so arranged to be able to sense the state of the sample on the action part.

(2) The device described in (1), which further comprises an output part for outputting a signal from a sensor.
(3) A system which comprises the device described in (2), and
a data processing unit connected to the output part of the device and for processing the signal from the sensor.
(4) A program for evaluating a state of a sample by a system containing a device and a data processing unit, wherein

the device comprises
an action part for allowing lactate dehydrogenase to act on a sample,
a sensor for sensing the state of a sample on which lactate dehydrogenase is allowed to act, which sensor is so arranged to be able to sense the state of the sample on the action part, and
an output part for outputting a signal from a sensor, wherein
a data processing unit is connected to an output part of the device and is constituted for processing a signal from the sensor,
the program causes the device to execute
the measurement processing in which the state of the sample on which lactate dehydrogenase is allowed to act at an action part is sensed and measured by a sensor and to convert to a signal, and
transfer processing for transferring the signal obtained in the measurement processing from an output part to the data processing unit, and
the program causes the data processing unit to execute the data processing for carrying out a predetermined processing on a signal obtained in the measurement processing.

(5) A method for evaluating a state of a sample, which uses the device described in (1) or (2), or the system described in (3).
(6) The method described in (5), wherein the sample is a lactic acid-containing composition containing body fluids, interstitial fluid, blood, urine, tears, sweat, saliva, skin, meat, eyeballs, cornea, gastric juice of human and non-human organisms, foods and drinks, brewed products, chemical products, water or a soil.
(7) The method described in (5) or (6), wherein the state of the sample is a physical state by an exercise load, a disease state, a brewed state of a brewed product accompanied by change in an amount of lactic acid, a degree of maturing and afterripening of a foods and drinks accompanied by change in an amount of lactic acid, a contained ratio of lactic acid in a production of a chemical product accompanied by change in an amount of lactic acid, water accompanied by change in an amount of lactic acid or an amount of lactic acid in a soil.
(8) A device for monitoring a state of a sample, wherein the device comprises an action part for allowing

(A) flavin-dependent lactate dehydrogenase which maintains about 20% or more of an initial activity when it is elapsed at 37°C for 10 days in a solution,
(B) flavin-dependent lactate dehydrogenase which maintains about 20% or more of an initial activity when it is elapsed at 37°C for 3 days or longer in a solution, or
(C) flavin-dependent lactate dehydrogenase which maintains about 20% or more of an initial activity when it is elapsed at 37°C for 15 hours or longer in a solution to act on a sample.

(9) A system which comprises the device described in (8) further containing an output part and the output part being connected to a data processing unit.
(10) A method for monitoring a state of a sample which uses the device described in (8) or the system described in (9).
(11) Lactate dehydrogenase which comprises an amino acid sequence at positions 110 to 502 in an amino acid sequence shown by SEQ ID NO:4 or an amino acid sequence having 70% or more of identity thereof, an amino acid sequence at positions 113 to 505 in an amino acid sequence shown by SEQ ID NO:7 or an amino acid sequence having 70% or more of identity thereof, an amino acid sequence or an amino acid sequence having 70% or more of identity thereof, an amino acid sequence at positions 112 to 503 in an amino acid sequence shown by SEQ ID NO:10 or an amino acid sequence having 70% or more of identity thereof, or an amino acid sequence at positions 102 to 499 in an amino acid sequence shown by SEQ ID NO:12 or an amino acid sequence having 70% or more of identity thereof.
(12) The lactate dehydrogenase described in (11), which has an amino acid sequence shown by SEQ ID NO:4, SEQ ID NO:7, SEQ ID NO:10 or SEQ ID NO:12, or an amino acid sequence having 70% or more of identity thereof.

(13) Nucleic acid encoding the lactate dehydrogenase described in (11) or (12).

(14) A host cell having the nucleic acid described in (13).

(15) A method for producing lactate dehydrogenase, which comprises culturing the host cell described in (14).

(16) A method for evaluating a state of a sample, which comprises,

i) a step of bringing the lactate dehydrogenase described in (11) or (12) into contact with a sample, and

ii) a step of measuring lactic acid.

EFFECTS OF THE INVENTION

According to the present invention, a device for evaluating a state of a sample, a method for evaluating a state of a sample and FMN-LDH used therefor can be provided.

BRIEF DESCRIPTION OF THE DRAWINGS

Fig. 1(a) is a schematic drawing of a sensor chip 10 which is one embodiment of the present invention, and (b) to (d) are schematic drawings showing members constituting the sensor chip 10.

Fig. 2 is a drawing showing residual activity ratios of PkLDH and ScLDH after heat treatment at each temperature.

Fig. 3A and Fig. 3B are drawings showing residual activity ratios of PkLDH, ScLDH, CaLDH and OgLDH after preserving at 37°C for each time.

Fig. 4 is a schematic drawing showing one example of the device and the system of the embodiment of the present invention.

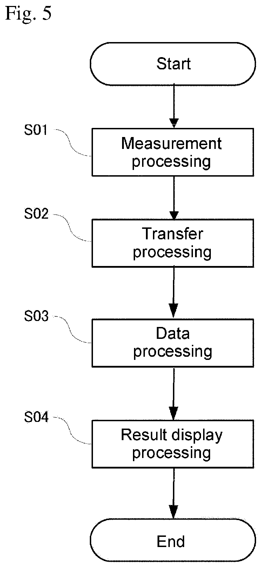

Fig. 5 is a flow chart showing one example of the program of the embodiment of the present invention.

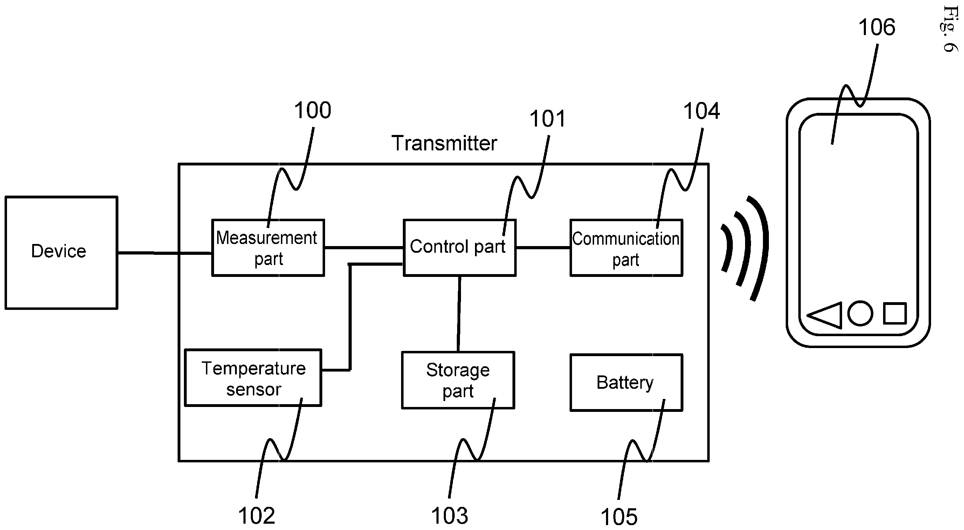

Fig. 6 is a schematic drawing showing one example of the device and the system utilizing the transmitter of the embodiment of the present invention Fig. 7 is a drawing showing a stable pH of PkLDH evaluated in Example 4.

Fig. 8 is a drawing showing an optimum pH of PkLDH evaluated in Example 4.

Fig. 9 is a drawing showing a relationship between a concentration of lactic acid and activity of PkLDH evaluated in Example 4.

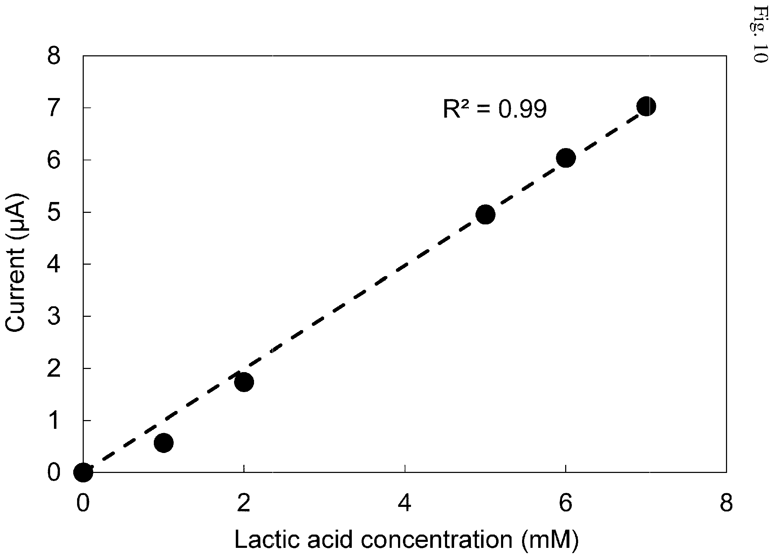

Fig. 10 is a drawing showing a relationship between a concentration of lactic acid and a response current value evaluated in Example 8.

Fig. 11-1 shows an alignment of an amino acid sequence of the lactate dehydrogenase of the present invention.

Fig. 11-2 is a continuation of Fig. 11-1.

Fig. 12 is a drawing showing residual activity ratios of PkLDH and N-terminal deleted mutants of PkLDH treated at 55°C for 15 minutes evaluated in Example 11.

Fig. 13 is a drawing showing residual activity ratios of PkLDH and monosubstituted mutants treated at 55°C for 15 minutes evaluated in Example 11.

EMBODIMENTS TO CARRY OUT THE INVENTION

Hereinafter, embodiments of the present invention will be specifically explained. Incidentally, the following embodiments are embodiments at the time of materializing the present invention, and do not limit the present invention to the scope.

The sample referred to in the present invention may be any object for measuring whether it contains lactic acid or not, and may be body fluids of humans and non-human organisms, for example, interstitial fluid, blood, urine, tears, sweat, saliva, skin, meat, eyeballs, cornea or gastric juice, or may be a lactic acid-containing composition including foods and drinks, brewed products or chemical products, which are not lactic acid fermented, lactic acid fermenting or lactic acid fermented. Further, it may be a material present in an environment such as water or soil containing lactic acid.

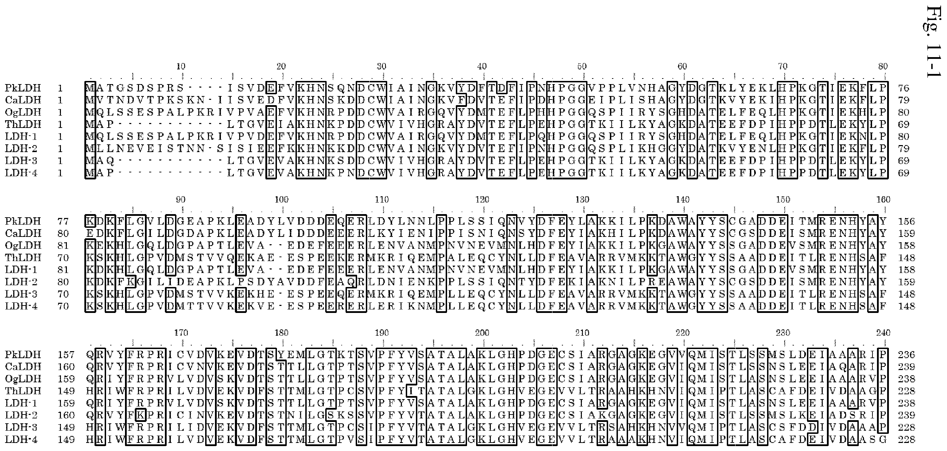

The lactate dehydrogenase (LDH) referred to in the present invention may be any enzyme which catalyzes a reversible reaction in which a pyruvate ion is reduced to a lactic acid ion using NADH which is a reducing agent, and has been known NAD-dependent lactate dehydrogenase and FMN-dependent lactate dehydrogenase, and is preferably FMN-dependent lactate dehydrogenase, more preferably lactate dehydrogenase having an amino acid sequence represented by SEQ ID NO:4, SEQ ID NO:7, SEQ ID NO:10, or SEQ ID NO:12 or an amino acid sequence having an identity of 70% or more, 75% or more, 80% or more, 85% or more, 90% or more, or 95% or more thereof. It is more preferably lactate dehydrogenase having the amino acid sequence at positions 97 to 502 in the amino acid sequence represented by SEQ ID NO:4 or the amino acid sequence having an identity of 70% or more, 75% or more, 80% or more, 85% or more, 90% or more, or 95% or more thereof. Alternatively, it is lactate dehydrogenase having the amino acid sequence at positions 100 to 505 in the amino acid sequence represented by SEQ ID NO:7 or the amino acid sequence having an identity of 70% or more, 75% or more, 80% or more, 85% or more, 90% or more, or 95% or more thereof. Or else, it is lactate dehydrogenase having the amino acid sequence at positions 99 to 503 in the amino acid sequence represented by SEQ ID NO:10 or the amino acid sequence having an identity of 70% or more, 75% or more, 80% or more, 85% or more, 90% or more, or 95% or more thereof. Alternatively, it is lactate dehydrogenase having the amino acid sequence at positions 89 to 499 in the amino acid sequence represented by SEQ ID NO:12 or the amino acid sequence having an identity of 70% or more, 75% or more, 80% or more, 85% or more, 90% or more, or 95% or more thereof. It is more preferably lactate dehydrogenase having the amino acid sequence at positions 110 to 502 in the amino acid sequence represented by SEQ ID NO:4 or the amino acid sequence having an identity of 70% or more, 75% or more, 80% or more, 85% or more, 90% or more, or 95% or more thereof. Or else, it is lactate dehydrogenase having the amino acid sequence at positions 113 to 505 in the amino acid sequence represented by SEQ ID NO:7 or the amino acid sequence having an identity of 70% or more, 75% or more, 80% or more, 85% or more, 90% or more, or 95% or more thereof. Or else, it is lactate dehydrogenase having the amino acid sequence at positions 112 to 503 in the amino acid sequence represented by SEQ ID NO:10 or the amino acid sequence having an identity of 70% or more, 75% or more, 80% or more, 85% or more, 90% or more, or 95% or more thereof. Or else, it is lactate dehydrogenase having the amino acid sequence at positions 102 to 499 in the amino acid sequence represented by SEQ ID NO:12 or the amino acid sequence having an identity of 70% or more, 75% or more, 80% or more, 85% or more, 90% or more, or 95% or more thereof. Incidentally, it is also possible to connect the amino acid sequence at positions 1 to 96 in SEQ ID NO:4, the amino acid sequence at positions 1 to 99 in SEQ ID NO:7, the amino acid sequence at positions 1 to 98 in SEQ ID NO:10, the amino acid sequence at positions 1 to 88 in SEQ ID NO:12 or the amino acid sequence having an identity of 70% or more, 75% or more, 80% or more, 85% or more, 90% or more, or 95% or more thereof may be connected to the amino acid sequence at positions 97 to 502 in SEQ ID NO:4. Similarly, the amino acid sequence at positions 1 to 96 in SEQ ID NO:4, the amino acid sequence at positions 1 to 99 in SEQ ID NO:7, the amino acid sequence at positions 1 to 98 in SEQ ID NO:10, the amino acid sequence at positions 1 to 88 in SEQ ID NO:12 or the amino acid sequence having an identity of 70% or more, 75% or more, 80% or more, 85% or more, 90% or more, or 95% or more thereof may be connected to the amino acid sequence at positions 100 to 505 in amino acid sequence SEQ ID NO:7, the amino acid sequence at positions 99 to 503 in SEQ ID NO:10 or the amino acid sequence at positions 89 to 499 in SEQ ID NO:12. Also, with regard to the region of the amino acid sequence at positions 1 to 96 in SEQ ID NO:4, the amino acid sequence at positions 1 to 99 in SEQ ID NO:7, the amino acid sequence at positions 1 to 98 in SEQ ID NO:10 or the amino acid sequence at positions 1 to 88 in SEQ ID NO:12, as shown in Fig. 11-1, sequence identity is low so that it can be said that importance in the lactate dehydrogenase of the present invention is low. Accordingly, among the full-length amino acid sequences, it is preferably lactate dehydrogenase containing the amino acid sequence at positions 97 to 502 in SEQ ID NO:4, the amino acid sequence at positions 100 to 505 in SEQ ID NO:7, the amino acid sequence at positions 99 to 503 in SEQ ID NO:10, the amino acid sequence at positions 89 to 499 in SEQ ID NO:12 or the amino acid sequence having identity of 70% or more, 75% or more, 80% or more, 85% or more, 90% or more, or 95% or more thereof, and with regard to the region containing the amino acid sequence at positions 1 to 96 in SEQ ID NO:4, the amino acid sequence at positions 1 to 99 in SEQ ID NO:7, the amino acid sequence at positions 1 to 98 in SEQ ID NO:10 or the amino acid sequence at positions 1 to 88 in SEQ ID NO:12, the sequence identity may be sufficient to have 45% or more, 50% or more, 60% or 70% or more, respectively, or this region may be deleted. Alternatively, it may be partially deleted and, for example, 1 to 2, 3, 4, 5, 6, 7, 8, 9, 10 or 15 amino acids may be deleted from the N-terminal sequence. For example, the amino acids at positions 2 to 10 in SEQ ID NO:4 may be deleted. When it is deleted, methionine can be optionally added to the beginning of the sequence.

(Regarding homology region)

The identity or similarity of the amino acid sequence can be calculated by a program such as maximum matching or search homology of GENETYX Ver. 11 (manufactured by GENETYX CORPORATION), etc., or a program such as maximum matching or multiple alignment, etc., of DNASIS Pro (manufactured by Hitachi Solutions, Ltd.). In order to calculate the identity of the amino acid sequence, when two or more LDHs are aligned, the positions of the amino acids that are the same in the two or more LDHs. Based on such information, the same region in the amino acid sequence can be determined.

In addition, it is also possible to investigate the positions of amino acids that are similar in two or more LDHs. For example, a plurality of amino acid sequences can be aligned using CLUSTALW, and in this case, Blosum62 is used as an algorithm, and amino acids judged to be similar when a plurality of amino acid sequences are aligned may be referred to as similar amino acids. In the mutants of the present invention, amino acid substitutions can be due to substitutions between such similar amino acids. According to such alignment, it is possible to investigate the positions occupied by regions and similar amino acids having the same amino acid sequence with regard to a plurality of amino acid sequences. Based on such information, homology region (conservation region) in the amino acid sequence can be determined.

For example, based on the lactate dehydrogenase represented by SEQ ID NO:4, the positions 138 to 140, 150 to 154, 185 to 194, 217 to 222, 243 to 245, 271 to 278, 280 to 283, 355 to 367, 398 to 401, 403 to 406, 425 to 433, 447 to 450, and 455 to 457 can correspond to the homology region. In addition, for example, based on the lactate dehydrogenase represented by SEQ ID NO:4, the positions 138 to 140, 150 to 154, 185 to 188, 191 to 194, 217 to 219, 243 to 245, 271 to 275, 280 to 283, 355 to 366, 398 to 400, 405 to 410, 425 to 431, 447 to 450, and 455 to 457 can correspond to the homology region. Moreover, the amino acid sequence in the homology region of the lactate dehydrogenase of the present invention has sequence identity of 75% or more with the amino acid sequence in the homology region of SEQ ID NO:4, for example, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, and for example, 99% or more.

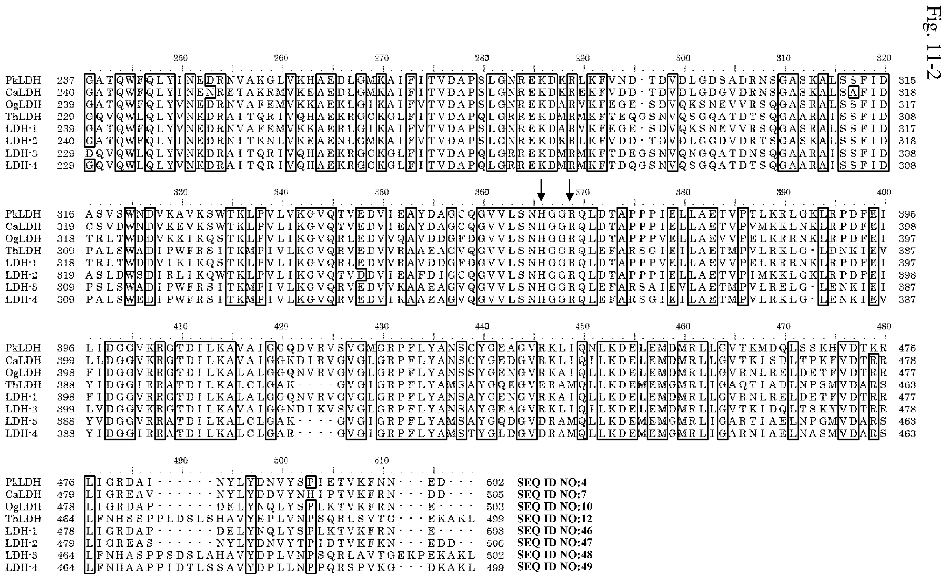

Further, as an amino acid particularly important for the lactate dehydrogenase of the present invention to maintain its activity, there may be mentioned histidine at position 361 and arginine at position 364 in lactate dehydrogenase represented by SEQ ID NO:4. It is also possible to clarify the position corresponding to these important amino acid positions in lactate dehydrogenase other than SEQ ID NO:4 by using the alignment of the amino acid sequences of each lactate dehydrogenase shown in Figs. 11-1 and 11-2 (the position shown by the arrow in Fig. 11-2). Incidentally, in Figs. 11-1 and 11-2, in addition to the lactate dehydrogenase represented by SEQ ID NO:4, SEQ ID NO:7, SEQ ID NO:10, or SEQ ID NO:12, it shows alignments including 503 amino acids (LDH-1) represented by SEQ ID NO:46 in which positions 2 to 51 are deleted among 558 amino acids which are the amino acid sequences of Ogataea polymorpha-derived lactate dehydrogenase, 506 amino acids (LDH-2) represented by SEQ ID NO:47 in which positions 2 to 86 are deleted among 558 amino acids which are the amino acid sequences of Candida californica-derived lactate dehydrogenase, 502 amino acids (LDH-3) represented by SEQ ID NO:48 which is the amino acid sequences of Chaetomium globosum-derived lactate dehydrogenase, and 499 amino acids (LDH-4) represented by SEQ ID NO:49 which is the amino acid sequences of Madurella mycetomatis-derived lactate dehydrogenase.

First, the device and the system of the present invention will be explained.

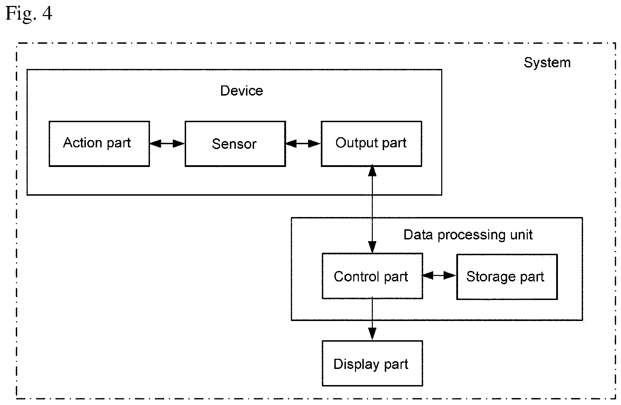

As shown in Fig. 4, the device for evaluating the state of the sample in the present invention includes an action part for allowing lactate dehydrogenase to act on a sample, and a sensor for sensing the state of the sample on which lactate dehydrogenase is allowed to act. The sensor is so arranged that it can sense the state of the sample at the action part. Also, the action part of the sensor may be included in a gel, preferably biocompatible gel. In this case, the gel is brought into contact with the sample, water containing lactic acid is collected through the gel by the effect of osmotic pressure, etc., to contact lactic acid with the sensor whereby the lactic acid in the sample can be sensed As a component (that is, a material of a gel constituting a gel structure) having biocompatibility, it is not particularly limited as long as it is a material having cell adhesion and biocompatibility, having high transparency and having hydrophilicity, and both of synthetic molecules and biomolecules can be used.

As the above-mentioned synthetic molecules, for example, there may be mentioned polyethylene glycol or a hydrophilic acrylic-based molecule such as acrylamide, etc. Among these, polyethylene glycol dimethacrylate is a stretchable polymer, so that it is preferable since it can improve not only affinity for cells and living tissues, but also stretchability, viscoelasticity and toughness.

Also, as the biomolecules, for example, there may be mentioned polysaccharides such as sodium alginate, etc., protein materials such as gelatin and silk, etc., and extracellular matrix such as collagen, etc. Among these, a material containing a large amount of protein is preferable. Accordingly, as the biocompatible gel material of the present embodiment, it is preferable to use silk fibroin gel as one of the components having biocompatibility. By using the silk fibroin gel containing a large amount of protein, the surface of the gel material has high biocompatibility, and when promotion of adhesiveness of the cells is observed, cytotoxicity can be reduced.

The above-mentioned synthetic molecules and biomolecules may be a polymer or a low molecule. Here, as the molecular weight (Mw) of the polymer, it is not particularly limited as long as the said polymer can constitute a gel structure, and for example, a polymer having a molecular weight of 5,000 to 1,000,000 Da or so can be used.

The action part for acting lactate dehydrogenase may be sufficient if it is constituted by a material suitable for the action, and refers to a member to which an enzyme is allowed to act and which is constituted by a material such as metal, plastic, cloth, liquid, paper and Nylon. Also, the sensor that senses the state of the sample may be integrated with a member on which the enzyme acts, may be separated therefrom, or may be connected directly or indirectly.

At the action part, a sample and the lactate dehydrogenase of the present invention are arranged. Also, if necessary, at the action part, an electron acceptor, and/or a reagent for showing change in the electron acceptor (mediator), etc., may be arranged. The sensor including the action part (it is sometimes expressed to as a working electrode) has a working electrode containing LDH of the present invention, a reference electrode and a counter electrode. As the working electrode, a carbon electrode, a gold electrode, a platinum electrode, etc., is used, and on the electrode, LDH of the present invention is immobilized. Further, or separately therefrom, an electron mediator may be immobilized on the working electrode. The counter electrode can be a conventional electrode such as a platinum electrode or Pt/C, etc. The reference electrode can be a conventional electrode such as an Ag/AgCl electrode, etc. As the immobilization method, there are a method using a crosslinking reagent, a method of encapsulating in a polymer matrix, a method of coating with a dialysis membrane, a photo-crosslinkable polymer, a conductive polymer, an oxidation-reduction polymer, etc., or it may be immobilized in a polymer together with an electron mediator which is represented by ferrocene or derivatives thereof or may be adsorbed and immobilized on the electrode, or these may be used in combination. Typically, after LDH of the present invention is immobilized on the carbon electrode using glutaraldehyde, it is treated with a reagent having an amine group to block glutaraldehyde. In place of glutaraldehyde, a crosslinking reagent such as poly(ethylene glycol)diglycidyl ether, etc., may be also used.

As the sensor that can be used in the device of the present invention, any sensor that can sense the state of the sample on which lactate dehydrogenase is allowed to act can be used without limitation.

[Sensor chip]

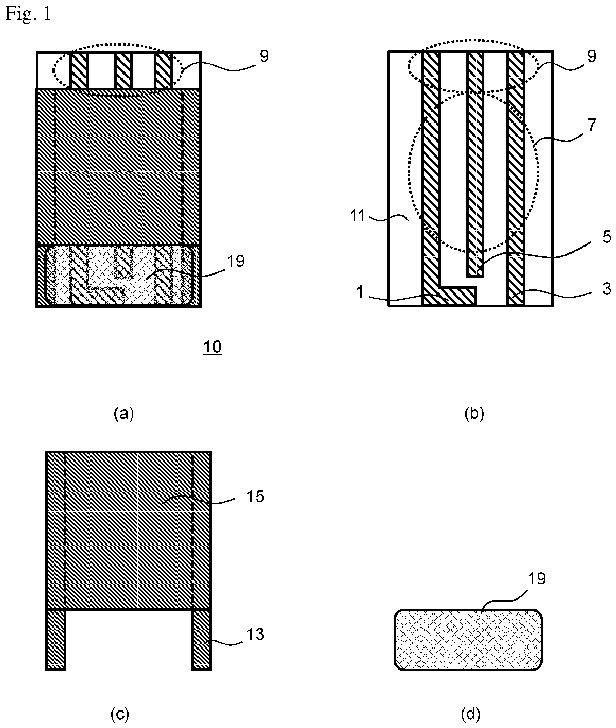

As the sensor, a sensor chip may be used. Fig. 1(a) is a schematic drawing of a sensor chip 10 regarding one embodiment of the present invention, and Fig. 1(b) to Fig. 1(d) are schematic drawings showing members constituting the sensor chip 10. The sensor chip 10 is provided with two or more electrodes arranged on a substrate 11. The substrate 11 is constituted by an insulating material. In Fig. 1(a) and Fig. 1(b), as an example, a working electrode 1, a counter electrode 3 and a reference electrode 5 are arranged on the substrate 11. Each electrode is electrically connected to a wiring portion 7, and the wiring portion 7 is electrically connected to a terminal 9 positioned to the opposite side of the wiring direction of each electrode. The working electrode 1, the counter electrode 3 and the reference electrode 5 are arranged apart from each other. Also, the working electrode 1, the counter electrode 3 and the reference electrode 5 are preferably constituted integrally with the wiring portion 7 and the terminal 9. Also, the counter electrode 3 and the reference electrode 5 may be an integrated type.

As shown in Fig. 1(a) and Fig. 1(c), a spacer 13 is arranged at the end of the substrate 11 parallel to the wiring part 7, and a cover 15 which covers the working electrode 1, the counter electrode 3, the reference electrode 5 and the spacer 13 is arranged. The spacer 13 and the cover 15 are constituted by an insulating material. It is preferable that the spacer 13 has a thickness substantially equal to those of the working electrode 1, the counter electrode 3 and the reference electrode 5, and is close contact with the working electrode 1, the counter electrode 3 and the reference electrode 5. In addition, the spacer 13 and the cover 15 may be constituted integrally. The cover 15 is a protective layer for preventing from deterioration of the wiring part 7 by being exposed to the outside air and short-circuiting due to penetration of the measurement sample.

As shown in Fig. 1(a) and Fig. 1(d), a reaction layer 19 is arranged on the working electrode 1, the counter electrode 3 and the reference electrode 5. The reaction layer 19 provides a field of reaction between lactic acid and lactate dehydrogenase. In one embodiment, the lactate dehydrogenase of the present invention may be coated, adsorbed or immobilized to these electrodes. It is preferable that the lactate dehydrogenase of the present invention is coated, adsorbed or immobilized to the working electrode. In another embodiment, the mediator may also be coated, adsorbed or immobilized to the electrode together with lactate dehydrogenase. As the electrode, a carbon electrode, and a metal electrode such as platinum, gold, silver, nickel and palladium, etc., can be used. In the case of the carbon electrode, there may be mentioned, as a material, pyrolytic graphite carbon (PG), glassy carbon (GC), carbon paste and plastic formed carbon (PFC), etc. The measurement system may be a two-electrode system or a three-electrode system, and for example, an enzyme can be immobilized on the working electrode. As the reference electrode, there may be mentioned a standard hydrogen electrode, a reversible hydrogen electrode, a silver-silver chloride electrode (Ag/AgCl), a palladium · hydrogen electrode and a saturated calomel electrode, etc., and from the viewpoints of stability and reproducibility, it is preferable to use Ag/AgCl.

Further, in order to reduce an amount of solution necessary for the measurement, a printing electrode may be used. In this case, it is preferable that the electrode is formed on the substrate 11 constituted by an insulating substrate. Specifically, it is desirable that the electrode is formed on the substrate 11 by a photolithography technique or a printing technique such as screen printing, gravure printing and flexographic printing, etc. Also, as a material of the insulating substrate, there may be mentioned silicon, glass, ceramic, polyvinyl chloride, polyethylene, polypropylene and polyester, etc., and it is preferable to use a material having strong resistance to various kinds of solvents and chemicals.

For example, in an action part in which potassium ferricyanide is contained as the electron acceptor, when lactate dehydrogenase is allowed to act on lactic acid contained in the sample, potassium ferricyanide is changed to potassium ferrocyanide. Potassium ferricyanide shows absorption at a wavelength of 420 nm so that the state of lactic acid in the sample can be sensed by measuring an absorbance at a wavelength of 420 nm with a spectrophotometer. Also, it is possible to measure the oxidation-reduction reaction electrochemically by a sensor when lactate dehydrogenase is allowed to act on lactic acid, and further potassium ferricyanide is allowed to act. As a sensor for subjecting to electrochemical measurement, specifically, for example, a sensor of Lactate Pro 2 model number LT-1730 manufactured by ARKRAY Inc., can be used. As the other electron acceptors, there may be used quinones, phenazines (for example, phenazine methosulfate), viologens, cytochromes (for example, cytochrome b, cytochrome c), phenoxazines, phenothiazines, ferricyanides, ferredoxins, ferrocene, osmium complex, ruthenium complex, phenylenediamines and derivatives thereof, etc. Incidentally, the lactate dehydrogenase of the present invention does not use oxygen as an electron acceptor.

For example, measurement of a concentration of lactic acid can be carried out as follows. A buffer is charged in a constant temperature cell and the cell is maintained at a constant temperature. As the working electrode, an electrode to which LDH and the electron acceptor (for example, a quinone-added polymer) are immobilized is used, and a counter electrode (for example, platinum electrode) and a reference electrode (for example, Ag/AgCl electrode) are used. A constant voltage is applied to a carbon electrode, and after the current becomes steady, a sample containing L-lactic acid is added and increase in the current is measured. In accordance with the calibration curve prepared by lactic acid solutions with standard concentrations, a lactic acid concentration in the sample can be calculated.

As a specific example, 4U LDH of the present invention is immobilized to a glassy carbon (GC) electrode, and a response current value with regard to the lactic acid concentration is measured. Into the electrolytic cell are added 1.8 ml of 50 mM potassium phosphate buffer (pH 7.5) and 0.2 ml of a 1 M potassium hexacyanoferrate (III) (potassium ferricyanide) aqueous solution are added. A GC electrode is connected to a potentiostat BAS100B/W (manufactured by BAS), the solution is stirred at 37°C, and +500 mV is applied to a silver · silver chloride (saturated KCl) reference electrode. To these systems is added 1 M L-lactic acid solution so as to have a final concentration of 1, 2, 3, 4, 5, 10, 20, 30, 40 or 50 mM, and a current value at a steady state is measured for each addition. This current value is plotted against a known lactic acid concentration (1, 2, 3, 4, 5, 10, 20, 30, 40, 50 mM) to prepare a calibration curve. According to this procedure, it is possible to determine lactic acid using an enzyme-immobilized electrode which uses FMN-LDH of the present invention.

In a certain embodiment, to a lactic acid sensor chip of the present invention is coated or immobilized 0.01 U to 1,000 U, 0.1 U to 1,000 U, more preferably 0.5 U to 700 U, more preferably 0.5 U to 500 U, more preferably 1 U to 300 U, more preferably 1 U to 100 U FMN-LDH solution of the present invention.

In a certain embodiment, a continuous lactic acid monitoring device having a lactic acid sensor of the present invention is provided. Also, in a certain embodiment, it is provided a method for continuously monitoring lactic acid during 14 days using LDH of the present invention. The term to be measured can be set as 5 seconds, 1 minute, 5 minutes, 1 hour, 2 hours, 6 hours, 12 hours, 15 hours, 24 hours, 2 days, 5 days, 7 days, 10 days, 14 days, etc., depending on the purpose.

In a certain embodiment, continuous lactic acid monitoring can be carried out with or without recalibration. In a certain embodiment, for example, recalibration can be carried out every 2, 3, 4, 5, 6, 7, 8, 9, 10, 11, 12, 13 or 14 days.

The lactate dehydrogenase of the present invention is sufficient in thermal stability. Therefore, simple and easy, and preferably continuous lactic acid monitoring can be realized.

The present invention also relates to a method for measuring lactic acid, which comprises i) bringing lactate dehydrogenase into contact with a sample and ii) measuring lactic acid. Specifically, the lactate dehydrogenase in step i) is FMN-dependent lactate dehydrogenase, more preferably lactate dehydrogenase having an amino acid sequence represented by SEQ ID NO:4, SEQ ID NO:7, SEQ ID NO:10, or SEQ ID NO:12 or an amino acid sequence having an identity of 70% or more, 75% or more, 80% or more, 85% or more, 90% or more, or 95% or more thereof. Also, specifically, the lactate dehydrogenase in step i) is lactate dehydrogenase having 0.01 U to 1,000 U, 0.1 U to 1,000 U, more preferably 0.5 U to 700 U, more preferably 0.5 U to 500 U, more preferably 1 U to 300 U, more preferably 1 U to 100 U. Also, the lactate dehydrogenase in step i) may be (A) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30, 35°C or 37°C for 10 days in a solution; (B) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30, 35°C or 37°C for 3 days or longer in a solution, or (C) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30, 35°C or 37°C for 15 hours or longer in a solution. Also, a temperature at the time of bringing the lactate dehydrogenase in step i) into contact with the sample may be sufficient to be 20 to 60°C, preferably in the range of 30 to 55°C, and preferably in the range of 30 to 40°C. Also, a pH at the time of bringing the lactate dehydrogenase in step i) into contact with the sample may be in the range of 3 to 10, preferably in the range of 5 to 9, and preferably in the range of 6 to 8.

Also, the step of measuring lactic acid in step ii) may be a single measurement, or may be a continuous measurement. A time for measurement in step ii) may be 5 seconds, 1 minute, 5 minutes, 1 hour, 2 hours, 6 hours, 12 hours, 15 hours, 24 hours, 2 days, 5 days, 7 days, 10 days or 14 days.

As shown in Fig. 4, the device for evaluating the state of the sample in the present invention may further contain an output part. The output part can output the signal from the sensor to the outside. Also, the output part may be connected to a data processing unit to form a system. That is, the system contains the device and the data processing unit. The data processing unit can process the signal from the sensor.

In the present specification, the "data processing unit" not only means a device such as a single computer, but also includes a concept of a network for processing information in the form of connecting other devices connected by a communication line such as a local network or the Internet.

Connection between the output part and the data processing unit means that the signal from the sensor is output from the output part and connected by an electric or electronic means so that it can be input to the data processing unit. For example, as a physical connection method for connection, in addition to the connection by wiring, it can also connect by a wireless communication such as a wireless LAN.

The data processing unit can contain a control part. The control part can be, for example, a CPU (Central Processing Unit). The program mentioned later can be read into the control part.

The data processing unit can contain a storage part. In the present specification, the "storage part" refers to a material that can be written so that the input information can be read such as a memory and a hard disk, etc. The storage part can be arranged inside a predetermined device. Also, the storage part can exist inside another device connected by a communication line such as a local network or the Internet. By containing the storage part, the signal measured by the sensor can be stored, and the result processed by the data processing unit can be stored.

The data processing unit can connect to the display part, which has a display part for displaying the result processed by the data processing unit, or can transfer to the display part. As the display part, a means for doing a known display such as a display, a printing device, an audio output device and output to the outside by a communication line such as a local network or the Internet can be used. That is, the display part not only physically has a display device in the data processing unit, but also displays the result to the other information processing device by transferring the data (result) to the other information processing device.

Also, the data processing unit can be equipped with a transmitter, and can be wirelessly transmitted and received to and from a separate measurement device equipped with a display part and a storage part. The transmitter is to measure the current value measured by the device at a measurement part 100, and transfers the current value to a control part 101. At the control part 101, the temperature in the vicinity of the device is measured by a temperature sensor 102, and after subjecting to temperature correction, the lactic acid concentration is calculated from the current value. At the control part 101, calculation of the lactic acid concentration is performed at predetermined sampling time intervals.

And at the control part 101, the calculated lactic acid concentration is subjected to integrated averaging at predetermined recording time intervals and records it in the storage part 103. The control part 101 transmits the value stored in the storage part 103 to a measurement device 106 via a communication part 104 in response to the instructions from the measurement device 106.

The transmitter in this embodiment has a built-in battery 105. The transmitter is so configured to be discarded at the time when the remaining battery level of the battery 105 becomes insufficient. The remaining battery level of the battery 105 is monitored through the measurement device 106. And at the time when the device is exchanged, the measurement device 106 is to confirm whether the remaining level of the battery 105 is sufficient or not until next exchange of the device to the remaining battery level of the battery 105 of the transmitter. And when if the remaining level is not sufficient, instruction is issued to the user to change the transmitter and to make the transmitter cannot be used.

According to the system of the present invention, it can evaluate the state of the sample, for example, a physical state by an exercise load, a disease state, a brewed state of a brewed product accompanied by change in an amount of lactic acid, a degree of maturing and afterripening of a foods and drinks accompanied by change in an amount of lactic acid, a content ratio of lactic acid in production of chemical products accompanied by change in an amount of lactic acid, a state of water or soil accompanied by change in an amount of lactic acid, etc.

The lactate dehydrogenase used in the device of the present invention may be (A) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30, 35°C or 37°C for 10 days in a solution; (B) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30, 35°C or 37°C for 3 days or longer in a solution, or (C) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30, 35°C or 37°C for 15 hours or longer in a solution. Also, the lactate dehydrogenase used in the device of the present invention may be flavin-dependent lactate dehydrogenase that maintains 70% or more activity even after 15 hours from the time applied for use.

The above-mentioned device may further contain an output part, and the output part may be connected to a data processing unit to constitute a system.

According to the device and the system of the present invention, evaluation or monitoring can be carried out non-invasively to the sample and can be measured directly without a step of sampling, that is, it is not a disposable measurement like inserting a test paper into the sensor and pressing a button, but it is extremely advantageous in the point that it can be used for a long period of time while it is still pasted or inserted.

Next, the program of the present invention will be explained. The program of the present invention is a program for evaluating the state of a sample by a system containing a device and a data processing unit.

In the present specification, the program can be an application software that causes a predetermined information processing device to execute a predetermined processing in order to operate a predetermined device. Also, the program of the present invention can also be a mobile application (app).

The program of the present invention is a program for operating the system as shown in the above-mentioned Fig. 4. The system contains a device and a data processing unit.

The device contains an action part, a sensor and an output part. With regard to the action part, the sensor and the output part, these are as explained above. That is, the action part is so constituted that it can be allowed to act lactate dehydrogenase on the sample. The sensor is so constituted that it is arranged to be capable of sensing the state of the sample to which lactate dehydrogenase is allowed to act at the action part. The output part is so constituted that it can output the signal from the sensor.

The data processing unit is connected to the output part of the device, and so constituted that the signal from the sensor is to be processed. The data processing unit can contain a control part, a storage part and a display part. With regard to the control part, the storage part and the display part, these are as explained above.

The program of the present invention causes the system to execute the measurement process S01, the transfer process S02 and the data process S03. The program of the present invention can further cause the system to execute the result display process S04.

In the measurement process S01, the program causes the system to execute a process so that the state of the sample on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process.

In the result display processing S04, the program causes the system to execute a process so that the predetermined display part displays the result processed by the data processing unit.

The present program can be specifically used as a program for the following uses.

The program of the present invention can be used as a program for the uses of food and beverage. Specifically, it is as follows.

(Embodiment 1 of program for food and beverage)

The program of the present embodiment can be a program for management of freshness of beef. That is, in the present embodiment, the sample is beef.

In the measurement process S01, the program causes the system to execute a process so that the state of the beef on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of freshness of beef.

In the result display processing S04, the program causes the system to execute a process so that the predetermined display part displays the result processed by the data processing unit. As a result, data in a data format suitable for management of freshness of beef can be displayed on the display part.

Incidentally, in the present embodiment, the expiration date of beef can be predicted by monitoring the temperature and humidity in addition to the value of the lactic acid. Also, by inputting the part of the meat as a parameter, the value of the meat can be calculated and used as an index of the selling price. Also, in the present embodiment, it is possible to propose the maturity of the meat according to the dish. In the present embodiment, the state of the meat can be sensed by the sensor by directly pasting the action part to beef.

Conventionally, it was the mainstream that management of freshness of beef was managed by a temperature. The lactate dehydrogenase of the present invention is sufficient in thermal stability, so that simple and easy, and continuous monitoring of lactic acid can be realized. Therefore, according to the program of the present embodiment, it can be made management of freshness using lactic acid as an index. Therefore, transportation conditions and storage conditions of beef can be appropriately carried out. By using the program of the present embodiment, it becomes an index objectively showing the value of beef so that it can support judgment of consumers.

(Embodiment 2 of program for food and beverage)

The program of the present embodiment can be a program for management of maturation and control index of beef. That is, in the present embodiment, the sample is beef.

In the measurement process S01, the program causes the system to execute a process so that the state of the beef on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of maturation and control index of beef.

In the result display processing S04, the program causes the system to execute a process so that the predetermined display part displays the result processed by the data processing unit. As a result, data in a data format suitable for management of maturation and control index of beef can be displayed on the display part.

Incidentally, in the present embodiment, the expiration date of beef can be predicted by monitoring the temperature, humidity and pH in addition to the value of the lactic acid. Also, by inputting the part of the meat as a parameter, the value of the meat can be calculated and used as an index of the selling price. Also, in the present embodiment, it is possible to propose the maturity of the meat according to the dish. In the present embodiment, the state of the meat can be sensed by the sensor by directly pasting the action part to beef.

Conventionally, it was the mainstream that management of maturation of beef was managed by a temperature. The lactate dehydrogenase of the present invention is sufficient in thermal stability, so that simple and easy, and continuous monitoring of lactic acid can be realized. Therefore, according to the program of the present embodiment, it can be made management of freshness using lactic acid as an index. Therefore, transportation conditions and storage conditions of beef can be appropriately carried out. By using the program of the present embodiment, it becomes an index objectively showing the value of beef so that it can support judgment of consumers.

(Embodiment 3 of program for food and beverage)

The program of the present embodiment can be a program for management of freshness of a fish such as tuna, etc. That is, in the present embodiment, the sample is a fish such as tuna, etc.

In the measurement process S01, the program causes the system to execute a process so that the state of the fish such as tuna, etc., on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of freshness of a fish such as tuna, etc.

In the result display processing S04, the program causes the system to execute a process so that the predetermined display part displays the result processed by the data processing unit. As a result, data in a data format suitable for management of freshness of a fish such as tuna, etc., can be displayed on the display part.

Incidentally, in the present embodiment, the degree of freshness and the expiration date of a fish such as tuna, etc., can be predicted by monitoring the temperature, humidity and K value in addition to the value of the lactic acid. In the present embodiment, also by inputting a kind of the fish as a parameter, the value of the fish can be calculated and used as an index of the selling price. Also, in the present embodiment, it is possible to propose the freshness of the fish according to the dish. In the present embodiment, the state of the fish such as tuna, etc., can be sensed by the sensor by directly pasting the action part to the eye of the fish, for example.

Conventionally, it was the mainstream that management of freshness of a fish such as tuna, etc., was managed by the temperature and K value (see "Sea-Nature and Culture", Bulletin of Tokai University, Faculty of Oceanography, Vol. 4, No. 2, pp. 31-46 (2006)). However, in the previous document, there are described that even when it is judged that the K value is low and freshness is high, the amount of lactic acid in the muscle is increased so that acid denaturation occurs, and as a result, freshness is sometimes low. The lactate dehydrogenase of the present invention is sufficient in thermal stability, so that simple and easy, and continuous monitoring of lactic acid can be realized. Therefore, according to the program of the present embodiment, it can be made management of freshness using lactic acid as an index. It can be also managed by the amount of lactic acid and the K value in combination. Therefore, transportation conditions and storage conditions of a fish such as tuna, etc., can be appropriately carried out. By using the program of the present embodiment, it becomes an index objectively showing the value of a fish such as tuna, etc., so that it can support judgment of consumers and wholesalers.

(Embodiment 4 of program for food and beverage)

The program of the present embodiment can be a program for management of maturation and control index of a fish such as tuna, etc. That is, in the present embodiment, the sample is a fish such as tuna, etc.

In the measurement process S01, the program causes the system to execute a process so that the state of the fish such as tuna, etc., on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of maturation and control index of a fish such as tuna, etc.

In the result display processing S04, the program causes the system to execute a process so that the predetermined display part displays the result processed by the data processing unit. As a result, data in a data format suitable for management of maturation and control index of a fish such as tuna, etc., can be displayed on the display part.

Incidentally, in the present embodiment, the maturation conditions of a fish such as tuna, etc., can be predicted by monitoring the temperature and humidity in addition to the value of the lactic acid. In the present embodiment, by inputting a kind of the fish as a parameter, the value of the matured fish can be calculated and used as an index of the selling price. Also, in the present embodiment, it is possible to propose the degree of maturation of the fish according to the dish. In the present embodiment, the state of the fish such as tuna, etc., can be sensed by the sensor by directly pasting the action part to the eye of the fish, for example.

Conventionally, it was the mainstream that management of maturation of a fish such as tuna, etc., was managed by the temperature, the maturation time, the temperature of a warm salt water to be used for thawing, and the water temperature. The lactate dehydrogenase of the present invention is sufficient in thermal stability, so that simple and easy, and continuous monitoring of lactic acid can be realized. Therefore, according to the program of the present embodiment, it can be made management of maturation using lactic acid as an index. Therefore, maturation conditions of a fish such as tuna, etc., can be appropriately carried out. By using the program of the present embodiment, it becomes an index objectively showing the value of a fish such as tuna, etc., so that it can support judgment of consumers and wholesalers.

(Other Embodiments of program for food and beverage)

The program of the present embodiment can be a program for food and beverage other than the above, for example, management of malolactic fermentation of wine, management of fermentation of Japanese rice wine, bread such as bagels, kuzumochi, management of fermentation of lactic acid of whisky, management of fermentation of tea, an index of safety of food, etc. According to the program of the present embodiment, management of fermentation of fermented liquor such as wine and Japanese rice wine, etc., which relies on experience and intuition, by making the pH value, dissolved oxygen value, etc., as indexes in addition to the value of lactic acid, appropriate temperature control or fermentation period can be obtained.

The program of the present invention can be used as a program for the use of animals. Specifically, it is as follows.

(Embodiment 1 of program for animals)

The program of the present embodiment can be a program for management of a race horse. That is, in the present embodiment, the sample is a race horse.

In the measurement process S01, the program causes the system to execute a process so that the state of a race horse on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of a race horse.

In the result display processing S04, the program causes the system to execute a process so that the predetermined display part displays the result processed by the data processing unit. As a result, data in a data format suitable for management of a race horse can be displayed on the display part.

Incidentally, in the training of race horses, a training program is set up using lactic acid in blood as an index. It has been known that an amount of lactic acid changes depending on the intensity of exercise, and when the balance between the amount of oxygen demand and the amount of supply during exercise is maintained, lactic acid in blood does not accumulate and when the balance of supply and demand is lost, it increases (JRA breeding ground diary, from training load using the lactic acid ground as an index). Accordingly, in the present embodiment, the aptitude of the training load of the race horse can be evaluated. Also, it is also possible to propose a training method of a horse using the obtained lactic acid value.

The lactate dehydrogenase of the present invention is sufficient in thermal stability, so that simple and easy, and continuous monitoring of lactic acid can be realized. Therefore, according to the program of the present embodiment, management of training, and management of health of race horses can be carried out. By attaching the sensor directly to the horse, or attaching to the surface of harness such as a saddle, etc., that comes into contact with the skin, and monitoring lactic acid contained in blood, skin or interstitial fluid, sweat, etc., it is also possible to reduce the burden on the trainer and the stress of the horse that blood is collected from the horse at each training and measured and analyzed.

(Embodiment 2 of program for animals)

The program of the present embodiment can be a program for measuring the degree of fatigue of a race horse. That is, in the present embodiment, the sample is a race horse.

In the measurement process S01, the program causes the system to execute a process so that the state of the race horse on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for measuring the degree of fatigue of a race horse.

In the result display processing S04, the program causes the system to execute a process so that the predetermined display part displays the result processed by the data processing unit. As a result, data in a data format suitable for measuring the degree of fatigue of a race horse can be displayed on the display part.

Incidentally, in the present embodiment, an amount of lactic acid changes depending on the degree of fatigue of a race horse, so that the degree of fatigue of the race horse can be predicted from the change in the amount of lactic acid.

The lactate dehydrogenase of the present invention is sufficient in thermal stability, so that simple and easy, and continuous monitoring of lactic acid can be realized. Therefore, according to the program of the present embodiment, an index of the degree of muscle fatigue of a race horse can be obtained, so that even a beginner can judge the degree of muscle fatigue of the race horse.

(Embodiment 3 of program for animals)

The program of the present embodiment can be a program for management of health of cattle, management of judging a shipping time or control of the quality of meat of beef cattle. That is, in the present embodiment, the sample is cattle.

In the measurement process S01, the program causes the system to execute a process so that the state of a cattle on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of health of cattle, management of judging a shipping time or control of the quality of meat of beef cattle.

In the result display processing S04, the program causes the system to execute a process so that the predetermined display part displays the result processed by the data processing unit. As a result, data in a data format suitable for management of health of cattle, management of judging a shipping time or control of the quality of meat of beef cattle can be displayed on the display part.

Incidentally, in the present embodiment, an ear is made as the action part and lactate dehydrogenase is allowed to act thereon, the state of a cattle can be sensed and measured by the sensor. In the ear, blood vessels pass through and an action part and a sensor are set up in the ear tag and arranged, it is possible to measure the blood of the ear as a specific sample. Sweat and skin can be also used as samples. Also, in the present embodiment, it is also possible to make stomach as an action part. For example, by measuring an amount of lactic acid in the blood, it is possible to suppress administration of grains containing lactic acid bacteria or lactic acid before suffering from acute acidosis.

In the present embodiment, it is also possible to carry out management of health, management of judging a shipping time or control of the quality of meat of beef cattle by a swallowing type sensor. That is, a digestive organ such as the stomach is made as an action part and lactate dehydrogenase is allowed to act thereon, the state of cattle can be sensed and measured by the sensor. In the present embodiment, depending on the amount of lactic acid of the cattle, the amount of lactic acid assimilated by the microorganisms existing in the stomach changes as a result, and according to the amount of lactic acid assimilated, volatile fatty acids are generated and the volatile fatty acids are absorbed by the cattle to become nutrients (carbon source) by the cattle. Accordingly, it is possible to predict the absorption efficiency of nutrients from change in an amount of lactic acid, prediction of health state of cattle, management of judging a shipping time or control of the quality of meat of beef cattle can be also carried out.

The lactate dehydrogenase of the present invention is sufficient in thermal stability, so that simple and easy, and continuous monitoring of lactic acid can be realized. Therefore, according to the program of the present embodiment, management of health of cattle, management of judging a shipping time or control of the quality of meat of beef cattle can be carried out.

(Embodiment 4 of program for animals)

The program of the present embodiment can be a program for management of health of a dairy cow. That is, in the present embodiment, the sample is a dairy cow.

In the measurement process S01, the program causes the system to execute a process so that the state of a dairy cow on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of health of a dairy cow.

In the result display processing S04, the program causes the system to execute a process so that the predetermined display part displays the result processed by the data processing unit. As a result, data in a data format suitable for management of health of a dairy cow can be displayed on the display part.

Incidentally, in the present embodiment, an ear is made as the action part and lactate dehydrogenase is allowed to act thereon, whereby the state of a dairy cow can be sensed and measured by the sensor. In the ear, blood vessels pass through and an action part and a sensor are set up in the ear tag and arranged, it is possible to measure the blood of the ear as a specific sample. Sweat and skin can be also used as samples. Also, in the present embodiment, it is also possible to make stomach as an action part. For example, by measuring an amount of lactic acid in the blood, it is possible to suppress administration of grains containing lactic acid bacteria or lactic acid before suffering from acute acidosis.

The lactate dehydrogenase of the present invention is sufficient in thermal stability, so that simple and easy, and continuous monitoring of lactic acid can be realized. Therefore, according to the program of the present embodiment, management of health of a dairy cow can be carried out. Also, by analyzing the component values of the milked milk together, the value of the obtained dairy products can be evaluated from the viewpoint of the final product and the health condition of dairy cows, so that it can support judgment of consumers and wholesalers.

The program of the present invention can be used as a program for the uses of plants. Specifically, it is as follows.

(Embodiment 1 of program for plants)

The program of the present embodiment can be a program for management of plants. That is, in the present embodiment, the sample a plant or soil. As the sample, it may be specifically a leaf, root, sap, fruit, seed, etc., of a plant, and may be a potted plant.

In the measurement process S01, the program causes the system to execute a process so that the state of plants on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for management of plants.

In the result display processing S04, the program causes the system to execute a process so that the predetermined display part displays the result processed by the data processing unit. As a result, data in a data format suitable for management of plants can be displayed on the display part.

If there is lactic acid in a soil, water absorption in plants is promoted. Accordingly, depending on the amount of lactic acid in the soil, water absorption of plants changes. Accordingly, in the present embodiment, the state of water absorption of plants can be evaluated.

The lactate dehydrogenase of the present invention is sufficient in thermal stability, so that simple and easy, and continuous monitoring of lactic acid can be realized. Therefore, according to the program of the present embodiment, it is possible to propose the timing of adding nutrients and watering the plants in the flowerpot. Further, if the pH is low, it is possible to suppress growth of various germs in plants. Accordingly, by measuring the pH together with the amount of lactic acid, it becomes possible to suppress growth of various germs.

(Embodiment 2 of program for plants)

The program of the present embodiment can be a program for indexes of management of a soil and management of hydroponic culture. That is, in the present embodiment, the sample is a soil or water.

In the measurement process S01, the program causes the system to execute a process so that the state of the soil on which lactate dehydrogenase is allowed to act at the action part is sensed and measured by the sensor to convert it to a signal.

In the transfer process S02, the program causes the system to execute a process so that the signal obtained in the measurement process is transferred from the output part to the data processing unit.

In the data process S03, the program causes the system to execute a process so that the data processing unit performs a predetermined processing on the signal obtained in the measurement process. The predetermined processing in the present embodiment is a processing for making a data format suitable for indexes of management of a soil, and management of hydroponic culture.

In the result display processing S04, the program causes the system to execute a process so that the predetermined display part displays the result processed by the data processing unit. As a result, data in a data format suitable for indexes of management of a soil, and management of hydroponic culture can be displayed on the display part.

If there is lactic acid in a soil and hydroponic water, water absorption in plants is promoted. Accordingly, depending on the amount of lactic acid in the soil and hydroponic water, water absorption of plants changes. Accordingly, in the present embodiment, the state of water absorption of plants existing in a soil and hydroponic water can be evaluated.

The lactate dehydrogenase of the present invention is sufficient in thermal stability, so that simple and easy, and continuous monitoring of lactic acid can be realized. Therefore, according to the program of the present embodiment, the timing of addition of nutrients or watering to plants, and the timing of topdressing can be proposed. Further, if the pH is low, it is possible to suppress growth of various germs of plants. Accordingly, by measuring the pH together with the amount of lactic acid, it becomes possible to suppress growth of various germs.

Conventionally, it was the mainstream that management of plants was management not based on data, such as adding fertilizer according to the time such as the season, etc., or adding water or nutrients when the condition of the plant deteriorates, etc. The lactate dehydrogenase of the present invention is sufficient in thermal stability, so that simple and easy, and continuous monitoring of lactic acid can be realized. When the lactic acid value contained in a soil becomes low, a system for calculating the amount of saccharides and lactic acid that can be assimilated by lactic acid bacteria can be provided in order to enhance the antibacterial property in the soil. On the contrary, when the lactic acid value becomes high, a system for calculating the kind and the amount of alkaline fertilizer to be added is provided. In addition, by measuring pH, electrical conductivity, amount of sunshine, temperature, humidity, and amount of amino acid, it is possible to construct and provide an accurate plant breeding program. Also, by analyzing the component values (for example, sugar content, acidity) of final products such as tomato, etc., with regard to the value of the obtained final products, an objective evaluation can be done from the viewpoint that cannot be seen from the consumers whether how the final products and plants have been grown so that it can support judgment of consumers and wholesalers.

The present invention also relates to lactate dehydrogenase having an amino acid sequence with an identity of 70% or more, 75% or more, 80% or more, 85% or more, 90% or more, or 95% or more as the amino acid sequence represented by SEQ ID NO:4, SEQ ID NO:7, SEQ ID NO:10, or SEQ ID NO:12, and nucleic acids encoding it. In a certain embodiment, the present invention is to provide DNA having a base sequence which has the sequence identity of 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 90%, 90%, 91%, 92%, 93%, 94%, 95%, 96%, 97%, 98% or 99% or more as the base sequence represented by SEQ ID NO:5, SEQ ID NO:8, SEQ ID NO:11, or SEQ ID NO:13, and encoding a protein having lactic acid activity.

(LDH gene)

In order to obtain a gene encoding LDH, a commonly used cloning method of gene is used. For example, chromosomal DNA or mRNA can be extracted from microbial cells having LDH-producing ability or various cells by a conventional method, for example, the method described in Current Protocols in Molecular Biology (WILEY Interscience, 1989). Further, cDNA can be synthesized using mRNA as a template. Using the chromosomal DNA or cDNA thus obtained, a library of the chromosomal DNA or cDNA can be prepared.

Next, a method in which, based on the above-mentioned amino acid sequence of LDH, an appropriate probe DNA is synthesized, and using this, the LDH gene is selected from the library of the chromosomal DNA or cDNA, or based on the above-mentioned amino acid sequence, an appropriate primer DNA is prepared, the DNA containing the objective gene fragment for encoding LDH is amplified by an appropriate polymerase chain reaction (PCR method) such as the 5'RACE method or the 3'RACE method, etc., and these DNA fragments are ligated to obtain DNA containing the full length of the objective LDH gene.

These LDH genes may be linked or inserted into various kinds of vectors, or may be integrated into chromosomes or genomes. When a vector is used, for cloning into the vector, a commercially available kit such as TA Cloning Kit (Invitrogen Corporation) or In-Fusion HD Cloning Kit (Clontech Laboratories, Inc.), etc.; a commercially available plasmid vector DNA such as pUC119 (TAKARA BIO INC.), pUC18 (TAKARA BIO INC.), pBR322 (TAKARA BIO INC.), pBluescript SK+ (Stratagene Corporation), pYES2/CT (Invitrogen Corporation), etc.; a commercially available bacteriophage vector DNA such as λEMBL3 (Stratagene Corporation), etc., can be used. By using the recombinant DNA, a host organism, for example, *Escherichia coli* (Escherichia coli), preferably *Escherichia coli* JM109 strain (TAKARA BIO INC.) or *Escherichia coli* DH5α strain (TAKARA BIO INC.) is transformed. Recombinant DNA contained in the obtained transformant is purified using QIAGEN Plasmid Mini Kit (QIAGEN N.V.), etc. For subjecting to mass production, it is preferable to use, as a host to produce LDH, a host organism transformed using a recombinant DNA containing the LDH gene, for example, *Escherichia coli,* yeast, mold cells, filamentous fungi, etc.

The present invention also relates to a method for producing lactate dehydrogenase, including a host cell having the above-mentioned nucleic acids and culturing the same. For producing the LDH using the strain having a producing ability of LDH excellent in stability obtained as mentioned above, this strain may be cultured by a usual solid culture method, but it is preferable to culture the same employing a liquid culture method as far as possible.

Also, as a medium for culturing the above-mentioned strain, for example, those in which one or more kinds of inorganic salts such as sodium chloride, monopotassium phosphate, dipotassium phosphate, magnesium sulfate, magnesium chloride, ferric chloride, ferric sulfate or manganese sulfate, etc., is added to one or more kinds of nitrogen sources such as yeast extract, tryptone, peptone, meat extract, corn steep liquor or soybean or leachate of wheat bran, etc., and further, if necessary, saccharine material, vitamin, etc., is/are appropriately added thereto, are used.

Incidentally, it is suitable that the initial pH of the medium is adjusted to pH 7 to 9.

Also, the culture may be carried out using any conditions, and for example, it can be carried out at an incubation temperature of 20 to 42°C, preferably at an incubation temperature of around 30°C for 4 to 24 hours, more preferably at an incubation temperature of around 30°C for 8 to 16 hours, by aerated and agitated deep culture, shaking culture, static culture, etc.

After completion of the culture, for collecting LDH from the culture, it can be obtained by a usual enzyme collection means. For example, the cells are subjected to ultrasonic destruction treatment, grinding treatment, etc., by a conventional method, or this enzyme is extracted using a lytic enzyme such as lysozyme, etc., or bacteriolysis is carried out by shaking or allowing to stand in the presence of toluene, etc., to discharge this enzyme out of the cells. And, this solution is filtered, centrifuged, etc., to remove the solid portion, and if necessary, the nucleic acid is removed by streptomycin sulfate, protamine sulfate or manganese sulfate, etc., and then, ammonium sulfate, alcohol, acetone, etc., are added thereto and fractionated, and the precipitate is collected to obtain a crude enzyme of LDH.

In order to further obtain an LDH purified enzyme preparation from the above-mentioned crude enzyme of LDH, for example, a gel filtration method using Sephadex, Superdex or Ultrogel, etc.; an adsorption elution method using an ion exchanger; an electrophoresis method using polyacrylamide gel, etc.; an adsorption elution method using hydroxyapatite; a precipitation method such as a sucrose density gradient centrifugation method, etc.; an affinity chromatography method; a fractionation method using molecular sieve membrane or hollow fiber membrane, etc., are appropriately selected, or carried out in combination of these to obtain a purified LDH enzyme preparation. In this way, LDH in which the desired stability has been improved can be obtained.

The lactate dehydrogenase of the present invention may be (A) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30 to 37°C for 10 days in a solution; (B) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30 to 37°C for 3 days or longer in a solution, or (C) flavin-dependent lactate dehydrogenase which maintains about 20% or more, 30% or more, 40% or more, 50% or more, 60% or more, 70% or more, 80% or more, 90% or more or 95% or more of an initial activity when it is elapsed at about 30 to 37°C for 15 hours or longer in a solution. Also, the lactate dehydrogenase of the present invention may be flavin-dependent lactate dehydrogenase that maintains 70% or more activity even after 15 hours from the time applied for use.

As a buffer material (buffer) which can be used for the reaction solution of LDH, for example, there may be mentioned buffer agent such as a borate buffer agent including boric acid and/or a salt thereof, a tris-hydrochloride buffer agent, a phosphate buffer agent including phosphoric acid and/or a salt thereof, for example, a potassium phosphate buffer agent or a sodium phosphate buffer agent, an organic acid buffer agent including an organic acid buffer agent and/or a salt thereof, for example, a tricarboxylate buffer agent including a tricarboxylic acid buffer agent and/or a salt thereof, for example, a citrate buffer agent including citric acid and/or a salt thereof, a monocarboxylate buffer agent including a monocarboxylic acid buffer agent and/or a salt thereof, for example, an acetate buffer agent including an acetic acid buffer agent and/or a salt thereof, etc. Also, as a buffer agent which can be used in the kit, etc., of the present invention, there may be mentioned Good's buffer agent including ACES (N-(2-acetamido-2-aminoethanesulfonic acid), BES (N,N-bis(2-hydroxyethyl)-2-aminoethanesulfonic acid), Bicin (N,N-bis(2-hydroxyethyl)glycine), Bis-Tris(bis(2-hydroxyethyl)iminotris(hydroxymethyl)methane), CHES (N-cyclohexyl-2-aminoethanesulfonic acid), EPPS (4-(2-hydroxyethyl)-1-piperazinepropanesulfonic acid), HEPES (4-2-hydroxyethyl-1-piperazineethanesulfonic acid), HEPPSO (N-(hydroxyethyl)piperazine-N'-2-hydroxypropanesulfonic acid), MES (2-(N-morpholino)ethanesulfonic acid), MOPS (3-(N-morpholino)propanesulfonic acid), MOPSO (2-hydroxy-3-morpholinopropanesulfonic acid), PIPES (piperazine-N,N'-bis(2-ethanesulfonic acid)), POPSO (piperazine-1,4-bis(2-hydroxypropanesulfonic acid)), TAPS (N-tris(hydroxymethyl)-methyl-3-aminopropanesulfonic acid), TAPSO (3-[N-tris(hydroxymethyl)methyl-amino]-2-hydroxypropanesulfonic acid), TES (N-tris(hydroxymethyl)methyl-2-aminoethanesulfonic acid), Tricine (N-tris(hydroxymethyl)methylglycine) and/or a salt thereof, etc. As a temperature at which LDH is reacted, it may be 20 to 60°C, and preferably may be within the range of 30 to 55°C. As a pH which LDH is reacted, it may be within the range of 3 to 10, and preferably may be within the range of 6 to 10.

EXAMPLES

Hereinafter, the present invention will be more specifically explained by Examples. However, the following Examples are intended only exemplification and are not intended to limit the technical scope of the present invention. Unless otherwise specifically mentioned, reagents are commercially available, or obtained or prepared according to the methods commonly used in this field of the art or procedures in the well-known literatures.

In the present invention, stability evaluation of FMN-LDH after heat treatment and evaluation of stability tests under various kinds of storage conditions were carried out according to the method of the following text examples unless otherwise specifically mentioned.

Example 1

(1) Preparation of recombinant plasmid pKK223-3-ScLDH DNA and pKK223-3-PkLDH DNA

As described in Biochem. J. 258, 255-259 (1989), the gene (including the stop codon TAA) of 1521 bp represented by SEQ ID NO:2 which encodes 506 amino acids represented by SEQ ID NO:1 in which amino acids at positions 2 to 85 among 591 amino acid which is an amino acid sequence of Saccharomyces cerevisiae-derived lactate dehydrogenase (ScLDH) have been removed was obtained as cDNA by PCR which is a usual method of gene fragments. Subsequently, 578 amino acids represented by SEQ ID NO:3 which are an amino acid sequence of Pichia kudriavzevii-derived lactate dehydrogenase (PkLDH) and ScLDH are compared, and the gene (including the stop codon TAA) of 1509 bp represented by SEQ ID NO:5 which encodes 502 amino acids represented by SEQ ID NO:4, in which at positions 2 to 77 have been removed, was obtained as cDNA by PCR which is a usual method of gene fragments. A DNA construct in which the ScLDH gene or the PkLDH gene, which are the target genes, was inserted into the multi-cloning site of a plasmid pKK223-3 by a conventional method was prepared. Specifically, the ScLDH gene or the PkLDH gene was linked according to the protocol attached to the kit using the In-Fusion HD Cloning Kit (manufactured by Clontech Laboratories, Inc.) at the In-Fusion Cloning Site existing in the multi-cloning site of pKK223-3 to obtain plasmids (pKK223-3-ScLDH and pKK223-3-PkLDH) for expression. Further, *Escherichia coli* JM109 was transformed using these plasmids, *Escherichia coli* JM109 (pKK223-3-ScLDH) strain and *Escherichia coli* JM109 (pKK223-3-PkLDH) strain were inoculated to 3 ml of an LB-amp medium [1% (w/v) BACTO tryptone, 0.5% (w/v) peptone, 0.5% (w/v) NaCl, 50 μg/ml ampicillin], and subjected to shake culture at 37°C for 16 hours to obtain each culture.

These cultures were collected by centrifugation at 10,000 × g for 1 minute to obtain bacterial cells. From this bacterial cells, recombinant plasmid pKK223-3-ScLDH and pKK223-3-PkLDH were extracted using GenElute Plasmid Miniprep Kit (manufactured by Sigma-Aldrich) to obtain 2.5 μg of recombinant plasmids pKK223-3-ScLDH and pKK223-3-PkLDH DNA.

(2) Production of LDH

*Escherichia coli* BL21 (pKK223-3-ScLDH) strain into which pKK223-3-ScLDH had been transduced was cultured in 3 ml of an LB-amp medium to which IPTG had been added so as to have a final concentration of 0.1 mM at 25°C for 24 hours. Similarly, *Escherichia coli* BL21 (pKK223-3-PkLDH) strain into which pKK223-3-PkLDH had been transduced was cultured in 3 ml of an LB-amp medium to which IPTG had been added so as to have a final concentration of 0.1 mM at 37°C for 24 hours. Each of the obtained cultured cells was washed with 10 mM potassium phosphate buffer (pH 7.5), and then, suspended in the same buffer, subjected to ultrasonic crushing treatment and centrifuged at 20,000 × g for 10 minutes to prepare 0.6 ml of a crude enzyme solution containing ScLDH or PkLDH.

(3) Measurement of activity of LDH

Using the above-mentioned crude enzyme solution containing ScLDH or PkLDH, oxidation activity to L-lactic acid was measured by the method shown in the following activity measurement method. LDH of the present invention catalyzes the reaction of oxidizing L-lactic acid to produce pyruvic acid. This may be sometimes referred to as LDH activity for convenience.

LDH activity of PkLDH of the present invention utilizes this principal of action, and for example, it can be measured using a measurement system using potassium ferricyanide as an electron acceptor.

(Reaction)        L-lactic acid + potassium ferricyanide → pyruvic acid+ potassium ferrocyanide

The degree of disappearance of the above-mentioned "potassium ferricyanide" is detected as the amount of change in absorbance at a wavelength of 420 nm, and the enzyme activity can be obtained based on this amount of change.

Specifically, LDH activity can be measured according to the following procedure. 0.15 mL of 1 M potassium phosphate buffer (pH 7.5), 0.15 mL of 0.1 M L-lactic acid solution, 0.075 mL 30 mM potassium ferricyanide solution, and 1.075 mL ultrapure water were mixed and the mixture was maintained at 30°C for 5 minutes. Then, 0.05 mL of the enzyme sample solution is added to start the reaction. The absorbances at the time of starting the reaction and over time are measured, and the amount of decrease (ΔA420) at the absorbance of 420 nm per 1 minute with the progress of the enzymatic reaction is obtained and the LDH activity is calculated according to the following equation. At this time, the LDH activity is defined that the amount of enzyme that reduces 1 μmol of potassium ferricyanide per 1 minute in the presence of L-lactic acid with an enzyme concentration of 10 mM at 30°C is to be 1 U.

[Numerical equation 1]

$$
\begin{aligned}
\text{LDH activity (U/mL)} &= \{ (-1) \times (\Delta Abs420 - \Delta Abs420, \text{blank}) \times 1.5 \times Df \} / (1.01 \times 0.05 \times 1.0) \\
&= -29.7 \times (\Delta Abs420 - \Delta Abs420, \text{blank}) \times Df
\end{aligned}
$$

Incidentally, in the equation, 1.5 represents the liquid volume (mL) of the reaction reagent + enzyme reagent, 1.01 represents the mmol molecular absorbance coefficient ($cm^2/\mu mol$) under the present activity measurement conditions, 0.05 represents the liquid volume (mL) of the enzyme solution, 1.0 represents the optical path length (cm), ΔA420 blank represents the amount of decrease at the absorbance of 420 nm per 1 minute when 10 mM phosphate buffer solution (pH 7.5) is added thereto to start the reaction in place of the enzyme sample solution, and df represents the dilution factor.

Example 2

(Temperature stability)

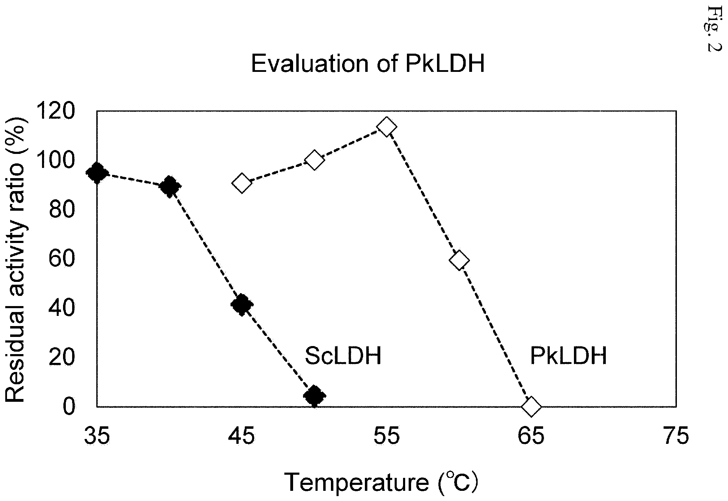

The above-mentioned crude enzyme solution was diluted so as to have a final concentration of 100 mM potassium phosphate buffer (pH 6.0) containing 0.07% BSA, and the temperature stability was examined. Specifically, the LDH enzyme solution prepared in (2) of Example 1 was diluted so as to have 6 U/ml, and after treating at each temperature (35°C, 40°C, 45°C, 50°C, 55°C, 60°C, 65°C) for 10 minutes, the LDH activity was measured, and the residual activity ratio was measured as compared with the LDH activity before the treatment. Also, for comparison, the same experiment was carried out on ScLDH. The results are shown in Fig. 2.

From the results, it could be found that ScLDH was unstable at 45°C, but PkLDH was stable after the heat treatment at 55°C.

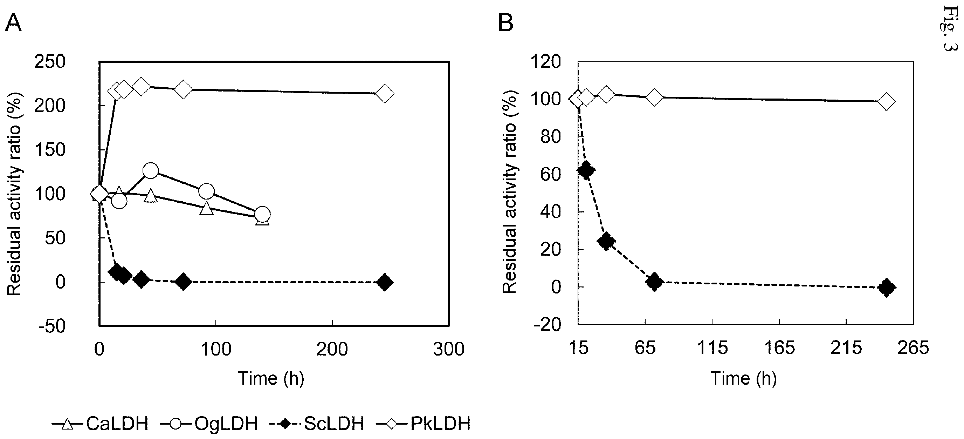

Subsequently, the long-term stability at 37°C was examined. Specifically, in the same manner as mentioned above, the LDH enzyme solution was diluted so as to have 6 U/ml with a buffer similarly in the test of the temperature stability and stored for each time. Thereafter, the residual activity ratio after each time elapsed was measured. Also, for comparison, a similar test was carried out on ScLDH. The results are shown in Fig. 3 (A and B). Fig. 3A is a graph of the residual activity ratio after each time elapsed, where the elapsed time is zero time is made 100%. According to the procedure, it could be found that, in PkLDH heated to a certain extent (in the present Example, 37°C), the residual activity ratio thereof is improved by a certain period of time during storage (Fig. 3A). Fig. 3B is a graph of the residual activity ratio after each time elapsed, when 15 hours from the start of the storage is set as the base time, where the activity ratio at the base time is made 100%. PkLDH retained 99% activity even after 245 hours elapsed from the start of the storage (after 230 hours from the base time), and after 15 hours elapsed, the residual activity ratio became constant, and even when it was stored at 37°C for about 10 days, it was not deactivated and was very stable (Fig. 3B). On the other hand, in ScLDH, the residual activity ratio began to decrease markedly from the start of the storage and the activity residual ratio after 21 hours (6 hours after the base time) was 62%, and the activity residual ratio after 65 hours (50 hours after the base time) was 0%, which was unstable. Also, even when the amount of the enzyme of ScLDH was evaluated to be 20 U/ml, stability was not significantly improved.

Example 3

(Purification of enzyme)

The crude enzyme solution of PkLDH obtained in (2) of Example 1 was applied to 1 ml of Q Sepharose Fast Flow resin (manufactured by GE healthcare) equilibrated with 20 mM potassium phosphate buffer (pH 7.5) to adsorb it to the resin. Protein that did not adsorb to the resin were dissolved out with the same buffer. Subsequently, it was washed by using 20 mM potassium phosphate buffer (pH 7.5) containing 50 mM sodium chloride and 20 mM potassium phosphate buffer (pH 7.5) containing 200 mM sodium chloride. Next, the adsorbed PkLDH was dissolved out using 20 mM potassium phosphate buffer (pH 7.5) containing 500 mM sodium chloride. The obtained crude enzyme solution of PkLDH was applied to HiLoad 26/10 Q Sepharose HP column (manufactured by GE healthcare) equilibrated with 20 mM potassium phosphate buffer (pH 7.5) containing 150 mM sodium chloride, and the buffer with a volume of one column was flown and the fraction(s) containing the target protein was/were recovered.

Further, the recovered enzyme solution was dialyzed by 10 mM potassium phosphate buffer (pH 7.5), adsorbed to Hiscreen Capto Q column (manufactured by GE healthcare) equilibrated with the same buffer, and then, PkLDH adsorbed to the resin was dissolved out and recovered by gradually increasing the NaCl concentration to 500 mM NaCl/10 mM potassium phosphate buffer (pH 7.5) with a gradient.

The obtained fractions were analyzed by SDS-PAGE, and confirmed to be purified to a purity free of other contaminating proteins, and made it as a purified sample of PkLDH. Incidentally, with regard to the thermal stability, it was equivalent to that of the crude enzyme solution.

Example 4

(pH stability)

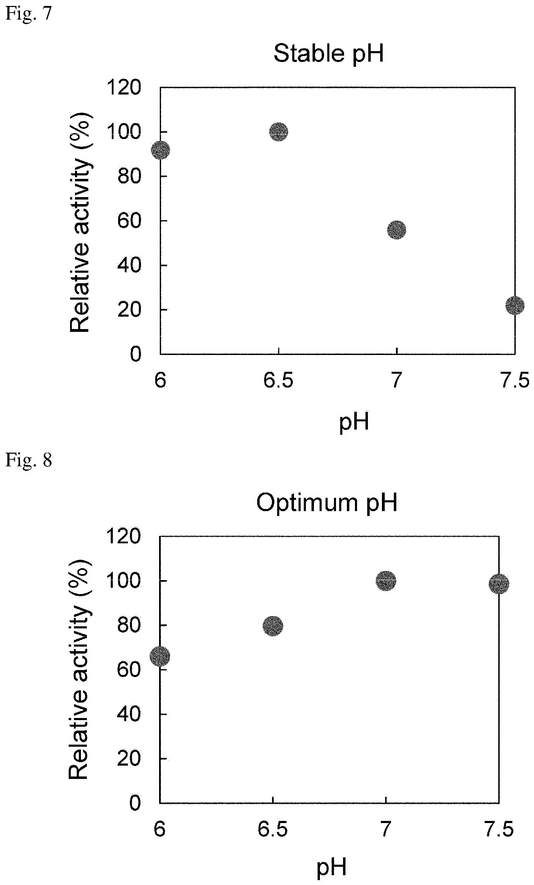

The pH stability was examined using the purified PkLDH enzyme solution (10 U/mL) obtained in Example 3. Using 100 mM potassium phosphate buffer (pH 6.0 to pH 7.5), after heat treatment at 55°C for 15 minutes, the enzyme was maintained in each buffer, and the activity was measured according to the activity measuring method described in (3) of Example 1 other than using each of the enzymes. The condition with the highest activity in the activity value after the treatment was set to 100%, and the relative value was obtained for the activity value under the other conditions. The results are shown in Fig. 7. As a result, it was the most stable at pH 6.5, and then, it was the relative activity (%) of 92% at pH 6.0.

(Optimum activity pH)

The optimum activity pH was examined using the purified PkLDH enzyme solution (10 U/mL) obtained in Example 3. Using 100 mM potassium phosphate buffer (pH 6.0 to pH 7.5), the enzymatic reaction was carried out at the respective pH at a temperature of 30°C, and the relative activity (%) was compared. The results are shown in Fig. 8. As a result, the optimum activity pH of the FMN-dependent lactate dehydrogenase of the present invention was shown to be the highest in the pH range of 6. 5 to 7. 5. Incidentally, it can be considered that there is a possibility of maintaining good activity even at pH 8.0.

(Determination of L-lactic acid)

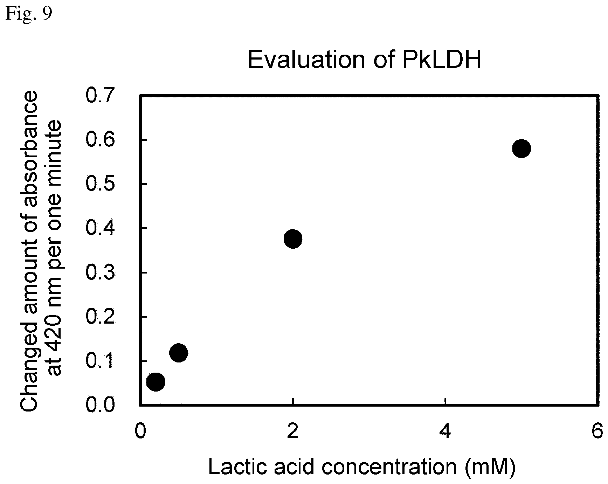

0.2 to 5 mM L-lactic acid was measured using the purified PkLDH enzyme solution (17 U/ml) obtained in Example 3. The results are shown in Fig. 9. As a result, the amount of change in absorbance per 1 minute at 420 nm was improved with the lactic acid concentration dependently, and it was shown that L-lactic acid could be determined.

(Oxidase activity)

With reference to the activity measurement method described in WO 2015/020200, the activity measurement was carried out using PkLDH as the enzyme and L-lactic acid being a final concentration of 10 mM as the substrate, and the measurement pH was made 7. 5. The oxidase activity was defined that the amount of the enzyme that produces 1 $\mu$mol of hydrogen peroxide per 1 minute in the presence of the substrate with a concentration of 10 mM as 1 unit (U). The amount of the enzyme to be used was an amount that shows 600 U/ml as the dehydrogenase activity. As a result, the oxidase activity of PkLDH was not detected. Accordingly, PkLDH was an enzyme that did not use oxygen as an electron acceptor.

Example 5

(Determination of L-lactic acid by printing electrode)

Determination of L-lactic acid by the printing electrode measurement is carried out using the purified PkLDH enzyme solution obtained in Example 3. Specifically, SCREEN-PRINTED ELECTRODES (manufactured by DropSens, product number DRP-110) on which a carbon working electrode and a silver reference electrode are printed is connected to ALS electrochemical analyzer 814D (manufactured by BAS) using a dedicated connector (manufactured by DropSens, DRP-CAC), and 5 $\mu$l of a PkLDH enzyme solution, 20 $\mu$l of 100 mM potassium phosphate buffer (pH 7.5) containing 1.5 M potassium chloride and 25 $\mu$l of a potassium ferricyanide aqueous solution are put on the electrode. And +400 mV (v.s. Ag/AgCl) of a voltage is applied thereto, and 5 $\mu$L of a lactic acid solution with a predetermined concentration is each put on the electrode to carry out the reaction, and the current value after 120 seconds is measured. As a result, lactic acid can be determined even in the electrochemical measurement.

Example 6

(4) Preparation of recombinant plasmid pKK223-3-CaLDH DNA and pKK223-3-OgLDH DNA and production of each LDH

Among 571 amino acids represented by SEQ ID NO:6 which are amino acid sequence of Candida inconspicua-derived lactate dehydrogenase (CaLDH), the gene (including the stop codon TAA) of 1518 bp represented by SEQ ID NO:8, which encodes 505 amino acids represented by SEQ ID NO:7 in which at positions 2 to 67 have been removed, was obtained as cDNA by PCR which is a usual method of gene fragments. Similarly, among 558 amino acids represented by SEQ ID NO:9 which are amino acid sequence of Ogataea parapolymorpha-derived lactate dehydrogenase (OgLDH), the gene (including the stop codon TAA) of 1512 bp represented by SEQ ID NO:11, which encodes 503 amino acids represented by SEQ ID NO:10 in which at positions 2 to 56 have been removed, was obtained as cDNA by PCR which is a usual method of gene fragments. In the same manner as in Example 1, a DNA construct in which the CaLDH gene or the OgLDH gene which is a target gene is inserted into the multi-cloning site of the plasmid pKK223-3 was prepared by a conventional method to obtain plasmids (pKK223-3-CaLDH and pKK223-3-OgLDH) for expression.

*Escherichia coli* BL21 (pKK223-3-CaLDH) strain into which pKK223-3-CaLDH had been transduced and *Escherichia coli* BL21 (pKK223-3-OgLDH) strain into which pKK223-3-OgLDH had been transduced were cultured in 3 ml of an LB-amp medium to which IPTG had been added so as to have a final concentration of 0.1 mM at 30°C for 24 hours. Each of the obtained cultured cells was washed with 10 mM potassium phosphate buffer (pH 7.5), and then, suspended in the same buffer, subjected to ultrasonic crushing treatment and centrifuged at 20,000 $\times$ g for 10 minutes to prepare 0.6 ml of a crude enzyme solution containing CaLDH or OgLDH.

Example 7

(Temperature stability)

In the same manner as in Example 2, the long-term stability at 37°C was examined. Provided that, 100 mM potassium phosphate buffer (pH 6.0) containing 0. 07% BSA as the final concentration was diluted so as to be CaLDH or OgLDH of about 20 U/ml, and stored for each time. Thereafter, the residual activity ratio after each time elapsed was measured. The results are shown in Fig. 3(A). CaLDH maintained 72% activity even after 140 hours elapsed from the start of storage, and OgLDH maintained 77% activity even after 140 hours elapsed from the start of storage, which were extremely stable as compared to that of ScLDH.

(pH stability)

Purification of CaLDH and OgLDH were carried out in the same manner as in PkLDH. Using the obtained purified enzyme solution (10 U/mL), pH stability was examined. As a result, both CaLDH and OgLDH were the most stable at pH 6.5, and showed a relative activity (%) of 80% or more in the range of pH 6.0 to 7.0.

(Oxidase activity)

When measurement of the oxidase activity of CaLDH and OgLDH was carried out, no oxidase activity was detected in either of them. Accordingly, CaLDH and OgLDH were enzymes that did not use oxygen as an electron acceptor.

Example 8

(5) Preparation of recombinant plasmid pKK223-3-ThLDH DNA and production of each LDH

The gene (including the stop codon TAA) of 1500 bp represented by SEQ ID NO:13, which encodes 499 amino acids represented by SEQ ID NO:12 which is an amino acid sequence of Thermothelomyces thermophilus-derived lactate dehydrogenase (ThLDH), was obtained as cDNA by PCR which is a usual method of gene fragments. In the same manner as in Example 1, a DNA construct in which the ThLDH gene which is a target gene is inserted into the multi-cloning site of the plasmid pKK223-3 by a conventional method was prepared to obtain a plasmid (pKK223-3-ThLDH) for expression.

Escherichia coli BL21 (pKK223-3-ThLDH) strain into which pKK223-3-ThLDH had been transduced was cultured in 3 ml of an LB-amp medium to which IPTG had been added so as to have a final concentration of 0.1 mM at 30°C for 24 hours. Each of the obtained cultured cells was washed with 10 mM potassium phosphate buffer (pH 7.5), and then, suspended in the same buffer, subjected to ultrasonic crushing treatment and centrifuged at 20,000 $\times$ g for 10 minutes to prepare 0.6 ml of a crude enzyme solution containing ThLDH.

Example 9

(Temperature stability)

In the same manner as in Example 2, the long-term stability at 37°C was examined. 100 mM potassium phosphate buffer (pH 6.0) containing 0.07% BSA as the final concentration was diluted so as to be ThLDH of about 20 U/ml, and stored for each time. Thereafter, the residual activity ratio after each time elapsed was measured. ThLDH maintained 74% activity even after 89 hours elapsed from the start of storage, which was extremely stable as compared to that of ScLDH. That is, when ThLDH is elapsed at 37°C for at least 3 days or longer, it can be said that it maintains 70% or more of the initial activity.

Example 10

(6) Preparation of PkLDH mutant

Construction of a mutant in which the N-terminal region of PkLDH was further deleted was carried out. First, in order to prepare a mutant (PkLDH-96) in which amino acids at positions 2 to 95 in SEQ ID NO:4 are deleted, using the recombinant plasmid pKK223-3-PkLDH obtained in Example 1 as a template, and synthetic oligonucleotides of SEQ ID NO:14 and 15, and KOD One PCR Master Mix (manufactured by TOYOBO CO., LTD.) were used, and the PCR reaction was carried out under the following conditions. That is, 10 μl of KOD One PCR Master Mix, 20 ng of pKK223-3-PkLDH which became a template, and each 6 pmol of the above-mentioned synthetic oligonucleotides were added, and the total amount was adjusted to 20 μl with sterilized water. The prepared reaction solution was subjected to a cycle of "98°C, 10 sec" - "55°C, 5 sec" - "68°C, 35 sec" which were repeated seven times using a thermal cycler (manufactured by Bio-Rad Laboratories, Inc.).

To the obtained solution containing the PCR product was added 1 μl of a restriction enzyme DpnI (manufactured by NEW ENGLAND BIOLABS), mixed and treated at 37°C for 30 minutes, and the remaining template DNA was cut. Subsequently, 2 μl of the obtained DpnI treated solution, 7 μl of sterilized water, 5 μl of Ligation high (manufactured by TOYOBO CO., LTD.), 1 μl of T4 Polynucleotide Kinase (manufactured by TOYOBO CO., LTD.) and 7 μl of deionized water were mixed, and reacted at 16°C for one hour. Using the reaction solution, *Escherichia coli* JM109 was transformed and developed on an LB-amp agar medium. In the same manner as in Example 1, the recombinant plasmid was extracted and purified to obtain 2.5 μg of DNA. The base sequence of DNA which encodes PkLDH-96 in the plasmid was determined using multi-capillary DNA analysis system Applied Biosystems 3130x1 Genetic Analyzer (manufactured by Life Technologies), and as a result, a DNA construct which encodes PkLDH-96 was obtained.

Similarly, in order to prepare a mutant (PkLDH-97) in which amino acids at positions 2 to 96 in SEQ ID NO:4 are deleted, synthetic oligonucleotides of SEQ ID NO:14 and 16, in order to prepare a mutant (PkLDH-98) in which amino acids at positions 2 to 97 in SEQ ID NO:4 are deleted, synthetic oligonucleotides of SEQ ID NO:14 and 17, in order to prepare a mutant (PkLDH-99) in which amino acids at positions 2 to 98 in SEQ ID NO:4 are deleted, synthetic oligonucleotides of SEQ ID NO:14 and 18, in order to prepare a mutant (PkLDH-100) in which amino acids at positions 2 to 99 in SEQ ID NO:4 are deleted, synthetic oligonucleotides of SEQ ID NO:14 and 19, in order to prepare a mutant (PkLDH-101) in which amino acids at positions 2 to 100 in SEQ ID NO:4 are deleted, synthetic oligonucleotides of SEQ ID NO:14 and 20, in order to prepare a mutant (PkLDH-102) in which amino acids at positions 2 to 101 in SEQ ID NO:4 are deleted, synthetic oligonucleotides of SEQ ID NO:14 and 21, in order to prepare a mutant (PkLDH-103) in which amino acids at positions 2 to 102 in SEQ ID NO:4 are deleted, synthetic oligonucleotides of SEQ ID NO:14 and 22, in order to prepare a mutant (PkLDH-104) in which amino acids at positions 2 to 103 in SEQ ID NO:4 are deleted, synthetic oligonucleotides of SEQ ID NO:14 and 23, in order to prepare a mutant (PkLDH-76) in which amino acids at positions 2 to 75 in SEQ ID NO:4 are deleted, synthetic oligonucleotides of SEQ ID NO:14 and 50, in order to prepare a mutant (PkLDH-84) in which amino acids at positions 2 to 83 in SEQ ID NO:4 are deleted, synthetic oligonucleotides of SEQ ID NO:14 and 51, in order to prepare a mutant (PkLDH-92) in which amino acids at positions 2 to 91 in SEQ ID NO:4 are deleted, synthetic oligonucleotides of SEQ ID NO:14 and 52, in order to prepare a mutant (PkLDH-110) in which amino acids at positions 2 to 109 in SEQ ID NO:4 are deleted, synthetic oligonucleotides of SEQ ID NO:14 and 53 were each used and PCR was carried out to obtain DNA constructs encoding PkLDH-76 to PkLDH-110.

In addition, using pKK223-3-PkLDH as a template, synthetic oligonucleotides of SEQ ID NO:24, 25, 26 and 27 were used and PCR was carried out. Specifically, using the recombinant plasmid pKK223-3-PkLDH as a template, synthetic oligonucleotides of SEQ ID NO:24 and 25, and KOD One PCR Master Mix (manufactured by TOYOBO CO., LTD.) were used, and the PCR reaction was carried out under the following conditions. That is, 10 μl of KOD One PCR Master Mix, 20 ng of pKK223-3-PkLDH which became a template, and each 6 pmol of the above-mentioned synthetic oligonucleotides were added, and the total amount was adjusted to 20 μl with sterilized water. The prepared reaction solution was subjected to a cycle of "98°C, 10 sec" - "55°C, 5 sec" - "68°C, 35 sec" which were repeated fifteen times using a thermal cycler (manufactured by Bio-Rad Laboratories, Inc.). The obtained PCR product was treated with the restriction enzyme DpnI, and after the remaining template DNA was cleaved, *Escherichia coli* JM109 was transformed and developed on an LB-amp agar medium. In the same manner as in Example 1, the recombinant plasmid was extracted and purified to obtain 2.5 μg of DNA. The base sequence of DNA which encodes the PkLDH mutant in the plasmid was determined using a multicapillary DNA analysis system Applied Biosystems 3130x1 Genetic Analyzer (manufactured by Life Technologies). Similarly, using the recombinant plasmid pKK223-3-PkLDH/L386R as a template, synthetic oligonucleotides of SEQ ID NO:26 and 27, and KOD One PCR Master Mix (manufactured by TOYOBO CO., LTD.) were used, the PCR reaction was carried out under the similar conditions to obtain 2.5 μg of DNA. The base sequence of DNA which encodes the PkLDH mutant in the plasmid was determined using a multicapillary DNA analysis system Applied Biosystems 3130x1 Genetic Analyzer (manufactured by Life Technologies), a DNA construct which encodes PkLDH/L386R/T461R/D464R, which is a mutant in which leucine at the position 386 of the amino acid sequence described in SEQ ID NO:4 is replaced with arginine, threonine at the position 461 is replaced with arginine, and aspartic acid at the position 464 is replaced with arginine was obtained. Incidentally, for example, "L386R/T461R/D464R" means that leucine at the position 386 of the amino acid sequence described in SEQ ID NO:4 is replaced with arginine, threonine at the position 461 is replaced with arginine, and aspartic acid at the position 464 is replaced with arginine, respectively, and further, the symbol "/" means that it has all the respective substitutions.

Also, using pKK223-3-PkLDH as a template, PCR was carried out using the synthetic oligonucleotides of SEQ

24

ID NO:28 and 29 to obtain a DNA construct which encodes PkLDH/F161L which is a mutant in which phenylalanine at the position 161 of the amino acid sequence described in SEQ ID NO:4 was replaced with leucine.

Also, using pKK223-3-PkLDH as a template, PCR was carried out using the synthetic oligonucleotides of SEQ ID NO:30 and 31 to obtain a DNA construct which encodes PkLDH/F187L which is a mutant in which phenylalanine at the position 187 of the amino acid sequence described in SEQ ID NO:4 was replaced with leucine.

Also, using pKK223-3-PkLDH as a template, PCR was carried out using the synthetic oligonucleotides of SEQ ID NO:32 and 33 to obtain a DNA construct which encodes PkLDH/F428L which is a mutant in which phenylalanine at the position 428 of the amino acid sequence described in SEQ ID NO:4 was replaced with leucine.

Also, using pKK223-3-PkLDH-97 as a template, PCR was carried out using the synthetic oligonucleotides of SEQ ID NO:32 and 33 to obtain a DNA construct which encodes PkLDH-97/F428L which is a mutant in which phenylalanine at the position 428 of the amino acid sequence described in SEQ ID NO:4 was replaced with leucine.

Also, using PkLDH-97/F428L as a template, PCR was carried out using the synthetic oligonucleotides of SEQ ID NO:34 and 35 to obtain a DNA construct which encodes PkLDH-97/F428L/L194A which is a mutant in which leucine at the position 194 of the amino acid sequence described in SEQ ID NO:4 in PkLDH-97/F428L was replaced with alanine.

Also, using PkLDH-97/F428L as a template, PCR was carried out using the synthetic oligonucleotides of SEQ ID NO:36 and 37 to obtain a DNA construct which encodes PkLDH-97/F428L/L222Y which is a mutant in which leucine at the position 222 of the amino acid sequence described in SEQ ID NO:4 in PkLDH-97/F428L was replaced with tyrosine.

Also, using PkLDH-97/F428L as a template, PCR was carried out using the synthetic oligonucleotides of SEQ ID NO:38 and 39 to obtain a DNA construct which encodes PkLDH-97/F428L/A274S which is a mutant in which alanine at the position 274 of the amino acid sequence described in SEQ ID NO:4 in PkLDH-97/F428L was replaced with serine.

Also, using PkLDH-97/F428L as a template, PCR was carried out using the synthetic oligonucleotides of SEQ ID NO:40 and 41 to obtain a DNA construct which encodes PkLDH-97/F428L/L277S which is a mutant in which leucine at the position 277 of the amino acid sequence described in SEQ ID NO:4 in PkLDH-97/F428L was replaced with serine.

Also, using PkLDH-97/F428L as a template, PCR was carried out using the synthetic oligonucleotides of SEQ ID NO:42 and 43 to obtain a DNA construct which encodes PkLDH-97/F428L/F313A which is a mutant in which phenylalanine at the position 313 of the amino acid sequence described in SEQ ID NO:4 in PkLDH-97/F428L was replaced with alanine.

Also, using PkLDH-97/F428L as a template, PCR was carried out using the synthetic oligonucleotides of SEQ ID NO:44 and 45 to obtain a DNA construct which encodes PkLDH-97/F428L/I314S which is a mutant in which isoleucine at the position 314 of the amino acid sequence described in SEQ ID NO:4 in PkLDH-97/F428L was replaced with serine.

In addition, similarly to the time of preparing the DNA construct which encodes PkLDH-97, in order to prepare a mutant (PkLDH-3/F428L) in which two amino acids in SEQ ID NO:4 are deleted, using PkLDH/F428L as a template, PCR was carried out using the synthetic oligonucleotides of SEQ ID NO:14 and 54, and as a result, a DNA construct which encodes PkLDH-3/F428L was obtained.

Also, in order to prepare a mutant (PkLDH-4/F428L) in which amino acids at position 2 to 3 in SEQ ID NO:4 were deleted, using PkLDH/F428L as a template, PCR was carried out using the synthetic oligonucleotides of SEQ ID NO:14 and 55, and as a result, a DNA construct which encodes PkLDH-4/F428L was obtained.

Also, in order to prepare a mutant (PkLDH-5/F428L) in which amino acids at 2 to 4 in SEQ ID NO:4 were deleted, using PkLDH/F428L as a template, PCR was carried out using the synthetic oligonucleotides of SEQ ID NO:14 and 56, and as a result, a DNA construct which encodes PkLDH-4/F428L was obtained.

Also, in order to prepare a mutant (PkLDH-6/F428L) in which amino acids at position 2 to 5 in SEQ ID NO:4 were deleted, using PkLDH/F428L as a template, PCR was carried out using the synthetic oligonucleotides of SEQ ID NO:14 and 57, and as a result, a DNA construct which encodes PkLDH-6/F428L was obtained.

Also, in order to prepare a mutant (PkLDH-7/F428L) in which amino acids at position 2 to 6 in SEQ ID NO:4 were deleted, using PkLDH/F428L as a template, PCR was carried out using the synthetic oligonucleotides of SEQ ID NO:14 and 58, and as a result, a DNA construct which encodes PkLDH-7/F428L was obtained.

Also, in order to prepare a mutant (PkLDH-8/F428L) in whichamino acids at position 2 to 7 in SEQ ID NO:4 were deleted, using PkLDH/F428L as a template, PCR was carried out using the synthetic oligonucleotides of SEQ ID NO:14 and 59, and as a result, a DNA construct which encodes PkLDH-8/F428L was obtained.

Also, in order to prepare a mutant (PkLDH-9/F428L) in which amino acids at position 2 to 8 in SEQ ID NO:4 were deleted, using PkLDH/F428L as a template, PCR was carried out using the synthetic oligonucleotides of SEQ ID NO:14 and 60, and as a result, a DNA construct which encodes PkLDH-9/F428L was obtained.

Also, in order to prepare a mutant (PkLDH-10/F428L) in which amino acids at position 2 to 9 in SEQ ID NO:4 were deleted, using PkLDH/F428L as a template, PCR was carried out using the synthetic oligonucleotides of SEQ ID NO:14 and 61, and as a result, a DNA construct which encodes PkLDH-10/F428L was obtained.

Also, in order to prepare a mutant (PkLDH-11/F428L) in which amino acids at position 2 to 10 in SEQ ID NO:4 were deleted, using PkLDH/F428L as a template, PCR was carried out using the synthetic oligonucleotides of SEQ ID NO:14 and 62, and as a result, a DNA construct which encodes PkLDH-11/F428L was obtained.

*Escherichia coli* BL21 strain into which the plasmids which encode the obtained various kinds of the PkLDH mutants had been transduced was cultured in 3 ml of an LB-amp medium to which IPTG had been added so as to have a final concentration of 0.1 mM at 30°C for 24 hours. Each of the obtained cultured cells was washed with 10 mM potassium phosphate buffer (pH 7.5), and then, suspended in the same buffer, subjected to ultrasonic crushing treatment and centrifuged at 20,000 × g for 10 minutes to prepare 0.6 ml of a crude enzyme solution containing various kinds of PkLDH mutants. Incidentally, with regard to 13 kinds of the mutants (PkLDH-76 to PkLDH-110) from which the N-terminal was deleted, the activity of crude enzyme solution was 0.3-fold to 6-fold as compared with that of the wild type PkLDH. Also, with regard to 9 kinds of the mutants (PkLDH-3/F428L to PkLDH-11/F428L) from which the N-terminal was deleted, the activity of crude enzyme solution was 2-fold to 11-fold as compared with that of PkLDH/F428L.

Example 11

(Temperature stability)

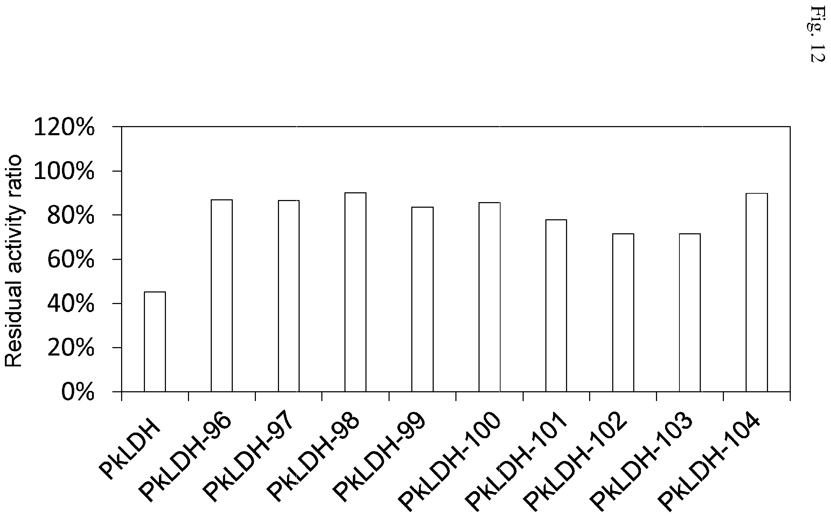

The crude enzyme solution (PkLDH-96 to PkLDH-104) prepared in Example 10 was diluted so as to be a 150 mM potassium phosphate buffer (pH 7.5) containing 0.15% BSA as the final concentration, and the temperature stability was examined. Specifically, the LDH enzyme solution which is the N-terminal deleted mutants prepared in Example 10 was diluted so as to be 6 U/ml, and after treating at 55°C for 15 minutes, the LDH activity was measured and the residual activity ratio was measured by comparing the LDH activity before treatment. Also, for comparison, the same test is also carried out for PkLDH. The results are shown in Fig. 12. It was found that PkLDH-96 to PkLDH-104 all had a higher residual activity ratio than that of PkLDH, so that thermal stability is high. Also, with regard to the other N-terminal deleted mutants (PkLDH-76, PkLDH-84, PkLDH-92, PkLDH-110, PkLDH-3/F428L, PkLDH-4/F428L, PkLDH-5/F428L, PkLDH-6/F428L, PkLDH-7/F428L, PkLDH-8/F428L, PkLDH-9/F428L, PkLDH-10/F428L, PkLDH-11/F428L), they were similarly treated at 50°C for 15 minutes. Subsequently, as reagents for measuring the LDH activity, 2,6-dichloroindophenol (DCIP) having a final concentration of 0.09 mM, phenazine methosulfate (PMS) having a final concentration of 0.5 mM, L-lactic acid having a final concentration of 10 mM and potassium phosphate buffer (pH 7.5) having a final concentration of 100 mM were prepared. To the 96-well plate were added 5 μL of the enzyme solution after the heat treatment and 145 μL of the reagents for measuring the LDH activity, and the mixture was incubated at 37°C for 5 minutes, the color of the reagent for measuring the activity was clearly changed to yellow. Accordingly, as compared with ScLDH which was completely inactivated by the heat treatment at 50°C, it was found that these mutants have high stability.

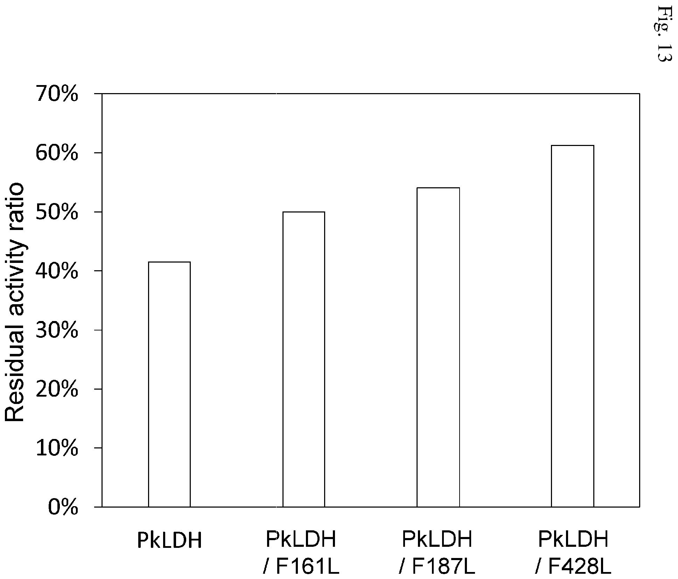

Subsequently, the crude enzyme solution (PkLDH/F161L, PkLDH/F187L, PkLDH/F428L) prepared in Example 10 was diluted so as to be 150 mM potassium phosphate buffer (pH 7.5) containing 0.15% BSA as a final concentration, and the temperature stability was examined. Specifically, the mono-substituted mutated LDH enzyme solution prepared in Example 10 was diluted so as to be 6 U/ml, and after treating at 55°C for 15 minutes, the LDH activity was measured, and it was compared with the LDH activity before the treatment and the residual activity ratio was measured. Also, for comparison, the same test is also carried out for PkLDH. The results are shown in Fig. 13. It was found that PkLDH/F161L, PkLDH/F187L and PkLDH/F428L all had higher residual activity ratio than that of PkLDH, so that thermal stability is high.

Also, using the crude enzyme solutions (PkLDH-97/F428L/L194A, PkLDH-97/F428L/L222Y, PkLDH-97/F428L/A274S, PkLDH-97/F428L/L277S, PkLDH-97/F428L/F313A, PkLDH-97/F428L/I314S) prepared in Example 10, they were treated at 50°C for 15 minutes. Subsequently, as the reagent for measuring the LDH activity, the system of the above-mentioned DCIP and PMS is used, and 5 μL of the enzyme solution after the heat treatment and 145 μL of the reagent for measuring the LDH activity measurement were added thereto, and the mixture was incubated at 37°C for 5 minutes, the color of the reagent for measuring the activity was clearly changed to yellow. Accordingly, as compared with ScLDH which was completely inactivated by the heat treatment at 50°C, it was found that these mutants have high stability. Incidentally, when measurement of the activity was similarly carried out using 1-methoxyphenazine methosulfate in place of PMS, similar results were obtained.

Next, in the same manner as in Example 2, the long-term stability at 37°C was examined. 100 mM potassium phosphate buffer (pH 6.0) containing 0.07% BSA as a final concentration, and various kinds of LDH mutants (PkLDH-96, PkLDH-97, PkLDH-104, PkLDH/L386R/T461R/D464R, PkLDH/F428L) were diluted so as to be about 20 U/ml, and stored for each time. As a result, PkLDH-96 maintained 95% activity even after 89 hours elapsed from the start of storage, PkLDH-97 maintained the activity of 114% after 43 hours elapsed, 114% after 72 hours elapsed, and 111% even after 171 hours elapsed from the start of storage, PkLDH-104 maintained the activity of 97% after 43 hours elapsed, 99% after 72 hours elapsed, and 88% even after 171 hours elapsed from the start of storage, PkLDH/L386R/T461R/D464R maintained 96% activity even after 89 hours elapsed from the start of storage, PkLDH/F428L maintained the activity of 99% after 43 hours elapsed, 107% after 72 hours elapsed, and 94% even after 171 hours elapsed from the start of storage, which were extremely stable as compared with that of ScLDH. That is, it can be said that these mutants maintain 70% or more of the initial activity when they are elapsed at 37°C for at least 3 days or longer. Also, with regard to PkLDH-98, PkLDH-99, PkLDH-100, PkLDH-101, PkLDH-102, PkLDH-103, PkLDH/F161L and PkLDH/F187L, thermal stability at 55°C is high as compared with that of PkLDH, so that there are high probability that they are extremely stable as compared with that of ScLDH in the long-term stability at 37°C.

Example 12

(Determination of L-lactic acid by PkLDH immobilized electrode)

Determination of L-lactic acid was carried out using an electrode to which the purified PkLDH enzyme solution obtained in Example 3 was immobilized. Specifically, 12 U of PkLDH was coated and dried on the working electrode of SCREEN-PRINTED ELECTRODES (manufactured by DropSens, product number DRP-C110) on which a carbon working electrode is printed. Subsequently, 3 $\mu$L of 2% poly(ethylene glycol)diglycidyl ether (Mn: 6,000, manufactured by Sigma) was coated and reacted at 4°C for 22 hours. It was washed with ultrapure water to make a PkLDH immobilized electrode. It was connected to ALS electrochemical analyzer 814D (manufactured by BAS) using a dedicated connector (manufactured by DropSens, DRP-CAC), and further, connected to a siler-silver chloride reference electrode and a platinum electrode, and three electrodes were immersed in 10 ml of PBS (pH 7.4) containing 0.1 mg/ml of Bindschedler's Green Leuco Base (manufactured by Tokyo Chemical Industry, Co., Ltd.). +200 mV (vs Ag/AgCl) was applied thereto, and the response current value when an L-lactic acid solution was added every fixed time period was recorded. The results are shown in Fig. 8. When 1 to 7 mM L-lactic acid was added, it was shown that the response current increased in a lactic acid concentration dependently, whereby L-lactic acid could be determined.

UTILIZABILITY IN INDUSTRY

According to a device for evaluating a state of a sample comprising an action part for allowing to act FMN-LDH on the sample and a sensor for sensing the state of the sample on which FMN-LDH is allowed to act, and a method for evaluating the state of the sample using the same of the present invention, lactic acid in a lactic acid-containing composition including interstitial fluid, blood, urine, tears, sweat, saliva, skin, meat, eyeballs, cornea, gastric juice of human and non-human organisms, foods and drinks, brewed product, chemical products, water, a soil can be measured stably for a long period of time, with good accuracy and simple and easily, so that it is useful for management of health of human and animals, or, management of manufacturing process and quality control of foods and drinks, brewed product, chemical products, etc.

EXPLANATION OF REFERENCE NUMERALS

| | |
|---|---|
| 1 | Working electrode |
| 3 | Counter electrode |
| 5 | Reference electrode |
| 7 | Wiring portion |
| 9 | Terminal |
| 10 | Sensor chip |
| 11 | Substrate |
| 13 | Spacer |
| 15 | Cover |
| 19 | Reaction layer |
| 100 | Measurement part |
| 101 | Control part |
| 102 | Temperature sensor |
| 103 | Storage part |
| 104 | Communication part |
| 105 | Battery |
| 106 | Measurement device |

**Claims**

**1.** A device for evaluating a state of a sample, which device comprises

an action part for allowing lactate dehydrogenase to act on the sample, and
a sensor for sensing the state of the sample on which lactate dehydrogenase is allowed to act, which sensor is so arranged to be able to sense the state of the sample on the action part.

2. The device according to Claim 1, which further comprises an output part for outputting a signal from a sensor.

3. A system which comprises

the device according to Claim 2, and
a data processing unit connected to the output part of the device and for processing the signal from the sensor.

4. A program for evaluating a state of a sample by a system containing a device and a data processing unit,

the device contains
an action part for allowing lactate dehydrogenase to act on a sample,
a sensor for sensing the state of a sample on which lactate dehydrogenase is allowed to act, which sensor is so arranged to be able to sense the state of the sample on the action part, and
an output part for outputting a signal from a sensor,
a data processing unit is connected to an output part of the device and is constituted for processing a signal from the sensor,
the program causes the device to execute
the measurement processing in which the state of the sample on which lactate dehydrogenase is allowed to act at an action part is sensed and measured by a sensor and to convert to a signal, and
transfer processing for transferring the signal obtained in the measurement processing from an output part to the data processing unit, and
the program causes the data processing unit to execute the data processing for carrying out a predetermined processing on a signal obtained in the measurement processing.

5. A method for evaluating a state of a sample, which uses the device according to Claim 1 or 2 or the system according to Claim 3.

6. The method according to Claim 5, wherein the sample is a lactic acid-containing composition containing body fluids, interstitial fluid, blood, urine, tears, sweat, saliva, skin, meat, eyeballs, cornea, gastric juice of human and non-human organisms, foods and drinks, brewed products, chemical products, water or a soil.

7. The method according to Claim 5 or 6, wherein the state of the sample is a physical state by an exercise load, a disease state, a brewed state of a brewed product accompanied by change in an amount of lactic acid, a degree of maturing and afterripening of a foods and drinks accompanied by change in an amount of lactic acid, a contained ratio of lactic acid in a production of a chemical product accompanied by change in an amount of lactic acid, water accompanied by change in an amount of lactic acid or an amount of lactic acid in a soil.

8. A device for monitoring state of a sample, wherein the device comprises an action part for allowing

(A) flavin-dependent lactate dehydrogenase which maintains about 20% or more of an initial activity when it is elapsed at 37°C for 10 days in a solution,
(B) flavin-dependent lactate dehydrogenase which maintains about 20% or more of an initial activity when it is elapsed at 37°C for 3 days or longer in a solution, or
(C) flavin-dependent lactate dehydrogenase which maintains about 20% or more of an initial activity when it is elapsed at 37°C for 15 hours or longer in a solution to act on a sample.

9. A system which comprises the device according to Claim 8 which further comprises an output part, and the output part is connected to a data processing unit.

10. A method for monitoring a state of a sample which uses the device according to Claim 8 or the system according to Claim 9.

11. A lactate dehydrogenase which comprises an amino acid sequence at positions 110 to 502 in an amino acid sequence shown by SEQ ID NO:4 or an amino acid sequence having 70% or more of identity thereof, an amino acid sequence at positions 113 to 505 in an amino acid sequence shown by SEQ ID NO:7 or an amino acid sequence having 70% or more of identity thereof, an amino acid sequence or an amino acid sequence having 70% or more of identity thereof, an amino acid sequence at positions 112 to 503 in an amino acid sequence shown by SEQ ID NO:10 or an amino acid sequence having 70% or more of identity thereof, or an amino acid sequence at positions 102 to 499 in an amino acid sequence shown by SEQ ID NO:12 or an amino acid sequence having 70% or more of identity thereof.

12. The lactate dehydrogenase according to Claim 11, which has an amino acid sequence represented by SEQ ID NO:4, SEQ ID NO:7, SEQ ID NO:10, or SEQ ID NO:12 or an amino acid sequence having 70% or more of identity thereof.

13. Nucleic acid which encodes lactate dehydrogenase according to Claim 11 or 12.

14. Host cells having nucleic acid according to Claim 13.

15. A method for producing lactate dehydrogenase, which comprises culturing the host cells according to Claim 14.

16. A method for evaluating a state of a sample, which comprises

    i) a step of bringing lactate dehydrogenase according to Claim 11 or 12 into contact with the sample, and
    ii) a step of measuring lactic acid.

Fig. 1

10

(a)

(b)

(c)

(d)

Fig. 2

Fig. 3

Fig. 4

Fig. 5

S01 — Measurement processing

S02 — Transfer processing

S03 — Data processing

S04 — Result display processing

Start → Measurement processing → Transfer processing → Data processing → Result display processing → End

Fig. 6

Fig. 7

Stable pH

Fig. 8

Optimum pH

Fig. 9

Evaluation of PkLDH

Fig. 10

Fig. 11-1

# Fig. 11-2

(protein sequence alignment of PkLDH, CalDH, OgLDH, ThLDH, LDH-1, LDH-2, LDH-3, LDH-4 corresponding to SEQ ID NO:4, SEQ ID NO:7, SEQ ID NO:10, SEQ ID NO:12, SEQ ID NO:46, SEQ ID NO:47, SEQ ID NO:48, SEQ ID NO:49)

Fig. 12

Fig. 13

## INTERNATIONAL SEARCH REPORT

International application No.

PCT/JP2021/006153

### A. CLASSIFICATION OF SUBJECT MATTER

C07K 14/00(2006.01)i; C12M 1/34(2006.01)i; C12N 5/10(2006.01)i; C12N 1/15(2006.01)i; C12N 1/19(2006.01)i; C12N 1/21(2006.01)i; C12N 15/53(2006.01)i; C12Q 1/00(2006.01)i; C12Q 1/32(2006.01)i; C12N 9/04(2006.01)i; G01N 33/52(2006.01)i; C12P 21/02(2006.01)i

FI: C12N15/53 ZNA; C12Q1/00 C; C12N1/19; C12N1/21; C12N5/10; C12P21/02 C; C12N1/15; G01N33/52 C; C12Q1/32; C07K14/00; C12N9/04 F; C12M1/34 E

According to International Patent Classification (IPC) or to both national classification and IPC

### B. FIELDS SEARCHED

Minimum documentation searched (classification system followed by classification symbols)
C12Q1/00; C12Q1/32

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/MEDLINE/EMBASE/BIOSIS; UniProt/GeneSeq; SwissProt/GeneSeq

### C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 10-513063 A (BOEHRINGER MANNHEIM GMBH) 15 December 1998 (1998-12-15) claims | 1-7<br>16<br>8-10 |
| X<br>Y<br>A | JP 2014-501502 A (KIMBERLY-CLARK WORLDWIDE, INC.) 23 January 2014 (2014-01-23) claims | 1-7<br>16<br>8-10 |

☒ Further documents are listed in the continuation of Box C.    ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 16 April 2021 (16.04.2021) | 27 April 2021 (27.04.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
| --- |
| PCT/JP2021/006153 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 8-327581 A (CO.RI.AL. S.C.P.A) 13 December 1996 (1996-12-13) claims, examples | 1-7<br>16<br>8-10 |
| X<br>Y<br>A | JP 4-346796 A (TOYO JOZO CO., LTD.) 02 December 1992 (1992-12-02) claims, examples | 1-7<br>16<br>8-10 |
| X<br>Y<br>A | JP 6-153995 A (SEKISUI CHEMICAL CO., LTD.) 03 June 1994 (1994-06-03) claims, paragraph [0018] | 1-7<br>16<br>8-10 |
| X<br>Y<br>A | JP 61-159164 A (KIEL, Jonathan L.) 18 July 1986 (1986-07-18) claims, page 3, upper left column, line 3 from the bottom to lower left column, the bottom line | 1-7<br>16<br>8-10 |
| X<br>Y<br>A | JP 58-175498 A (MERCK PATENT GMBH) 14 October 1983 (1983-10-14) claims, page 3, upper left column, line 3 from the bottom to lower left column, the bottom line | 1-7<br>116<br>8-10 |
| X<br>Y | WO 2015/017721 A1 (NOVOZYMES A/S) 05 February 2015 (2015-02-05) in particular, SEQ ID NO: 114 | 11-15<br>16 |
| X<br>Y | A0A4TOX365, July 31, 2019 uploaded, [retrieved 16 April 2021], Definition: Uncharacterized protein, Detabase Uniprot [online] see entire text | 11-15<br>16 |
| X<br>Y | JP 2009-517045 A (NATUREWORKS LLC) 30 April 2009 (2009-04-30) in particular, SEQ ID NO: 80 | 11-15<br>16 |
| X<br>Y | WO 2012/068236 A2 (DYADIC INTERNATIONAL (USA) INC.) 24 May 2012 (2012-05-24) in particular, SEQ ID NO: 552, 776 | 11-15<br>16 |

Form PCT/ISA/210 (continuation of second sheet) (January 2015)

| INTERNATIONAL SEARCH REPORT | International application No. |
|---|---|
| | PCT/JP2021/006153 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
     ☐ The additional search fees were accompanied by the applicant's protest and, where applicable, the payment of a protest fee.

     ☐ The additional search fees were accompanied by the applicant's protest but the applicable protest fee was not paid within the time limit specified in the invitation.

     ☐ No protest accompanied the payment of additional search fees.

Form PCT/ISA/210 (continuation of first sheet (2)) (January 2015)

**INTERNATIONAL SEARCH REPORT**
Information on patent family members

International application No.

PCT/JP2021/006153

| Patent Documents referred in the Report | Publication Date | Patent Family | Publication Date |
|---|---|---|---|
| JP 10-513063 A | 15 Dec. 1998 | US 6162615 A<br>claims<br>EP 804610 A1 | |
| JP 2014-501502 A | 23 Jan. 2014 | US 2012/0130195 A1<br>claims<br>EP 2643473 A2 | |
| JP 8-327581 A | 13 Dec. 1996 | US 5759796 A<br>claims<br>EP 744466 A2 | |
| JP 4-346796 A | 02 Dec. 1992 | (Family: none) | |
| JP 6-153995 A | 03 Jun. 1994 | (Family: none) | |
| JP 61-159164 A | 18 Jul. 1986 | EP 175577 A2<br>page 28, lines 5-13 | |
| JP 58-175498 A | 14 Oct. 1983 | US 4490465 A<br>claims, column 2,<br>line 56 to column 3 | |
| WO 2015/017721 A1 | 05 Feb. 2015 | EP 3027733 A1<br>Seq No. 114 | |
| JP 2009-517045 A | 30 Apr. 2009 | US 2009/0253189 A1<br>Seq No. 80<br>EP 1960516 A2 | |
| WO 2012/068236 A2 | 24 May 2012 | (Family: none) | |

Form PCT/ISA/210 (patent family annex) (January 2015)

**INTERNATIONAL SEARCH REPORT**

International application No.

PCT/JP2021/006153

<Continuation of Box No. III>

The inventions in claims 1 and 11 share the common technical feature of a "lactate dehydrogenase", but said technical feature does not make a contribution over the prior art in light of the disclosures of documents 1-7 and thus cannot be said to be a special technical feature.

Moreover, the invention-specifying matters of claim 11 are expressed as options, the options share the common technical feature of a "lactate dehydrogenase", but said technical feature does not make a contribution over the prior art in light of the disclosures of documents 1-7 and thus cannot be said to be a special technical feature.

Therefore, the claims are classified into the following five inventions.

(Invention 1) Claims 1-10
An invention comprising a device for evaluating the condition of a specimen using a lactate dehydrogenase

(Invention 2)
An invention comprising amino acid sequences at positions 110-502 in the amino acid sequence represented by SEQ ID NO: 4, or an amino acid sequence that shares at least 70% identity therewith, in claims 11-16

(Invention 3)
An invention comprising amino acid sequences at positions 113-505 in the amino acid sequence represented by SEQ ID NO: 7, or an amino acid sequence that shares at least 70% identity therewith, in claims 11-16

(Invention 4)
An invention comprising amino acid sequences at positions 112-503 in the amino acid sequence represented by SEQ ID NO: 10, or an amino acid sequence that shares at least 70% identity therewith, in claims 11-16

(Invention 5)
An invention comprising amino acid sequences at positions 102-499 in the amino acid sequence represented by SEQ ID NO: 12, or an amino acid sequence that shares at least 70% identity therewith, in claims 11-16

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019150649 A
- JP 2017100039 A
- WO 17163521 A
- JP 5593689 B
- JP 2018519507 A
- WO 2015020200 A

### Non-patent literature cited in the description

- The Japanese Clinical Practice Guidelines for Management of Sepsis and Septic Shock 2016. *PNAS,* 15 September 2015, vol. 112 (37), 11642-11647
- *Research journal of sports performance,* 2011, vol. 3, 31-48
- *Methods Enzymol.,* 1978, vol. 53, 238-56
- Sea-Nature and Culture. *Bulletin of Tokai University, Faculty of Oceanography,* 2006, vol. 4 (2), 31-46
- Current Protocols in Molecular Biology. WILEY Interscience, 1989
- *Biochem. J.,* 1989, vol. 258, 255-259